# EUROPEAN PATENT SPECIFICATION

(11) **EP 0 774 122 B1**
(45) Date of publication and mention of the grant of the patent: **19.08.2009**
(21) Application number: 96920325.6
(22) Date of filing: 20.05.1996
(51) Int. Cl.: G01N 33/68, G01N 1/30

(54) **MEROCYANINE DYE PROTEIN STAINS**
PROTEIN-FÄRBUNG MITTELS MEROCYANIN FARBSTOFFE
COLORATION DE PROTEINES PAR COLORANT A BASE DE MEROCYANINE

(30) Priority: 19.05.1995 US 444895
(43) Date of publication of application: 21.05.1997
(73) Proprietor: Molecular Probes, Inc., Eugene, OR 97402-9132 (US)
(72) Inventor: HAUGLAND, Richard, P., Eugene, OR 97402 (US); SINGER, Victoria, L., Eugene, OR 97405 (US); JONES, Laurie, Jeanne, Eugene, OR 97405 (US); STEINBERG, Thomas, H., Eugene, OR 97401 (US)
(74) Representative: Couchman, Jonathan Hugh
(86) International application number: PCT/US1996/007297
(87) International publication number: WO 1996/036882

(56) References cited:
- EP-A- 0 517 050
- EP-A- 0 517 055
- US-A- 4 424 201
- JOURNAL OF THE AMERICAN CHEMICAL SOCIETY, vol. 73, November 1951, DC US, pages 5326-5332, XP002013886 L. G. S. BROOKER ET AL.: "Studies in the cyanine dye series. XI. The merocyanines. " cited in the application
- JOURNAL OF ORGANIC CHEMISTRY, vol. 49, no. 14, 1984, EASTON US, pages 2546-2551, XP002013887 A. HASSNER ET AL.: "Charge-shift probes of membrane potential. Synthesis." cited in the application
- JOURNAL OF THE AMERICAN CHEMICAL SOCIETY, vol. 73, November 1951, DC US, pages 5350-5356, XP002013888 L. G. S. BROOKER ET AL.: "Color and constitution. XI. Anhydronium bases of p-hydroxystyryl dyes as solvent polarity indicators."
- GAFFNEY ET AL.: BIOCHIMICA ET BIOPHYSICA ACTA, vol. 1117, 1992, pages 321-325,

## Description

### FIELD OF THE INVENTION

The invention relates to staining of poly(amino acids), including peptides, polypeptides and proteins in solution, in gels and on solid supports, using merocyanine dyes.

### BACKGROUND

Detection and analysis of poly(amino acids) is of great importance in a multitude of diverse activities, ranging from basic research to enzyme production, forensics analysis and diagnostics.

Several methods have been utilized to detect proteins or other poly(amino acids) in electrophoresis gels, including colorimetic methods like COOMASSIE Brilliant Blue (CBB) staining and silver staining, as well as fluorescent staining, e.g. nile red. The invention possesses many advantages over known methods for staining poly(amino acids) on gels: Staining is rapid, simple, and relatively insensitive to poly(amino acid) composition. Visualization is possible without destaining, and the stained bands remain detectable for days. The dyes used in the current method are readily soluble and stable in aqueous staining solutions. In addition, the dyes exhibit large Stokes shifts. Finally, the present invention allows the detection of as little as 1 ng of poly(amino acid) per band, which is comparable to silver staining, with less hazard and expense, and is more than an order of magnitude better than CBB or nile red staining.

The invention can also be used to detect poly(amino acids) on filter membranes or other solid supports, or in solution. The use of this invention for staining poly(amino acids) in solution can also be used to quantitate poly(amino acids) with greater sensitivity than other known methods, including absorbance-based Lowry, Bradford, and bicinchoninic acid (BCA) methods, and fluorescence-based methods (e.g. nile red), and with a greater dynamic range (Table 3).

The preparation and characterization of merocyanine dyes useful for the present invention is well documented. A number of styryl merocyanine dyes (referred to as RH dyes) have been previously prepared for measuring electric potentials in cell membranes. A variety of other merocyanine dyes have been described for use in the photographic industry, without reference to their fluorescence properties. Merocyanine 540 avidly binds to and photosensitises certain cells, including leukemia cells, whereas other cells have a low affinity for the dye, and this differential affinity is used for the detection of leukemia cells as well as the extracorporeal purging of bone marrow grafts and the decontamination of blood products.

Gaffney et al, Biochimica et Biophysica Acta, 117(1992) 321-325, describe that serum inhibits the merocyanine 540-sensitised inactivation of cells and an analysis of the interactions of the dye with serum and serum components. Their analysis included the steps of applying to a chromatographic column mixtures of dye with serum, albumin or lipoprotein, and then assaying eluted fractions spectrophotometrically but did not include heating the mixtures. The merocyanine 450 was found to bind to the proteins, confirming that serum inhibits merocyanine 540-sensitised photoinactivation of cells by competing with cellular binding sites for dye molecules.

US-A-4424201 teaches a method for the detection of malignant leukocytic cells by contacting a fluid containing such cells with a merocyanine dye, whereafter the dye is incorporated into the malignant cells and detected, for example, by fluorescence methods.

EP-A-517050 and EP-A-517055 relate to protein error indicators. EP-A-517050, for example, teaches that protein error indicators are pH indicators which include an ionisable group whose pKa is displaced by the presence of protein. The indicators are impregnated into a test strip for determining the presence of albumin in urine. These two European specifications disclose the use as protein error indicators of 4-hydroxy-3',5'-dihalostyryl merocyanine dyes impregnated in an absorbant carrier.

The present invention, which requires the staining and detection of poly(amino acids) outside of the cellular milieu, is neither anticipated nor obvious from previous references.

### DESCRIPTION OF DRAWINGS

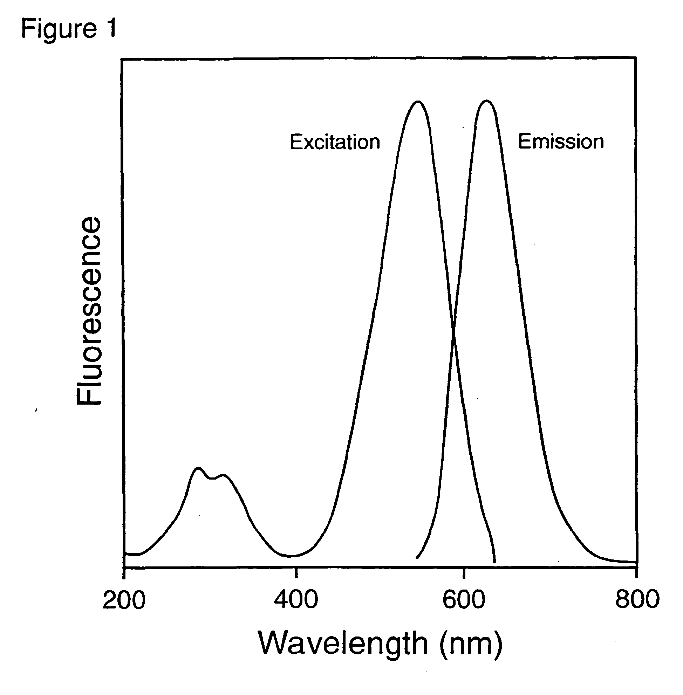

Figure 1: Normalized excitation and emission spectra in the presence of poly(amino acid). Fluorescence spectra were obtained of 1 µM of Dye 801, 150 µg/mL bovine serum albumin (BSA) and 0.05% SDS in 10 mM Tris-HCl, pH 7.5.

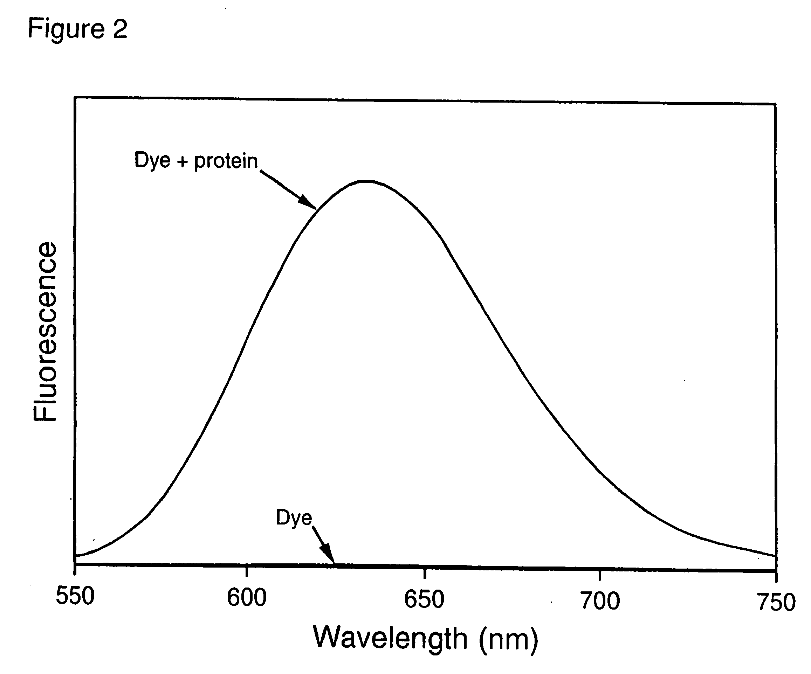

Figure 2: Fluorescence enhancement upon addition of poly(amino acid). Spectra were obtained of 1 µM Dye 801 and 0.05% SDS in 10 mM Tris-HCl, pH 7.5, in the absence and presence of 500 µg/mL BSA.
Figure 3: Correlation between poly(amino acid) concentration and fluorescence emission for Dye 307 as in Table 3, note 4, using 485 nm excitation and 590 nm emission.
Figure 4: Correlation between band fluorescence and total poly(amino acids) present in a gel band for Dye 801, as in Example 9.

### SUMMARY OF THE INVENTION AND DESCRIPTION OF PREFERRED EMBODIMENTS

Poly(amino acids) are stained according to the present invention by incubating the poly(amino acids) with one or more merocyanine dyes. As used herein, a poly(amino acid) is any homopolymer or heteropolymer of amino acids, including peptides, polypeptides, and proteins. The merocyanine dyes associate with the amino acid polymers either directly, or in the presence of a detergent, to yield both a strong colorimetric absorption and a strong fluorescence emission. Any poly(amino acid) thereby labeled can be detected with high sensitivity either in solution, or on a solid or semisolid support.

More specifically, the invention provides a method of detecting for a poly(amino acid) in a sample mixture, comprising the steps of:
a) preparing, from the sample mixture and a staining mixture that contains one or more merocyanine dyes, a cell-free combined mixture containing the poly(amino acid) (if present), the one or more merocyanine dyes and a detergent;
b) incubating the combined mixture to allow the dye in the staining mixture to associate with any poly(amino acid) in the sample mixture to form a dye-poly(amino acid) complex that gives a detectable optical response upon illumination;
c) illuminating a product obtained using step (b), e.g. dye-poly(amino acid) complex; and
d) observing the optical response.

Other aspects and embodiments of the invention are set forth in the claims.

### Selection of Dyes

Merocyanine dyes comprise a quaternary nitrogen heterocycle linked to an electron pair-donating moiety by an alkylene or polyalkylene bridge. A wide variety of electron pair-donating groups are known that stabilize the formally positive charge of the quaternary nitrogen heterocycle by resonance. Suitable electron pair-donating groups include dialkylaminophenyl, dialkylaminonaphthyl, electron-rich heterocycles and acyclic moieties containing electron pair-donating groups.

Preferred merocyanine dyes have the general formula

Q-B-M

wherein Q is a quaternized nitrogen heterocycle where the quaternizing group is a TAIL group, B is a covalent bridge that is an ethenyl or polyethenyl moiety, and M is an aromatic substituent or activated methylene substituent.

The covalent bridge, B, has the formula -(CR⁷=CR⁸)₈-, where R⁷ and R⁸ are independently H, C₁-C₆ alkyl or phenyl. Alternatively, R⁷ and R⁸ taken in combination complete a 5- or 6-membered saturated ring. The subscript n has a value of 1-3. Higher values of n shift the excitation and emission to longer wavelengths. Typically, R⁷ and R⁸ are both H. Preferably n = 1 or 2.

The quaternized nitrogen heterocycle Q is typically a substituted or unsubstituted pyridinium, quinolinium, benzoquinolinium or benzazolium moiety having the formula 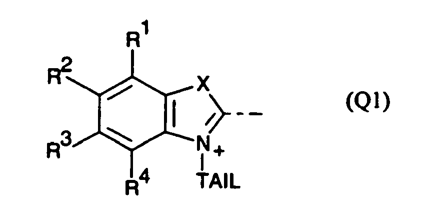 or 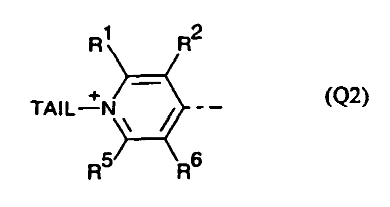 or the formula 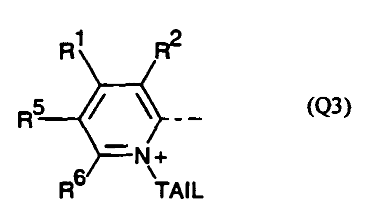 where the ring substituents R¹, R², R³ and R⁴ are optionally and independently H, Cl, F, C₁-C₆ alkyl, C₁-C₆ perfluoroalkyl, C₁-C₆ alkoxy, amino, or amino substituted by 1-2 C₁-C₆ alkyls. The ring substituents R⁵ and R⁶ are optionally and independently H, Cl, F, C₁-C₆ alkyl, C₁-C₆ perfluoroalkyl, C₁-C₆ alkoxy, amino, amino substituted by 1-2 C₁-C₆ alkyls, or phenyl. Alternatively, R⁵ and R⁶, when taken in combination, form a fused 6-membered aromatic ring (yielding a quinolinium moiety) that is optionally and independently substituted one or more times by Cl, F, C₁-C₆ alkyl, C₁-C₆ perfluoroalkyl, C₁-C₆ alkoxy, amino, or amino substituted by 1-2 C₁-C₆ alkyls. The quinolinium ring is optionally substituted by an additional fused 6-membered aromatic ring (yielding a naphtho-substituted pyridinium, or a benzoquinoline), that is also optionally and independently substituted one or more times by Cl, F, C₁-C₆ alkyl, C₁-C₆ perfluoroalkyl, C₁-C₆ alkoxy, amino, or amino substituted by 1-2 C₁-C₆ alkyls. Typically, R⁵ and R⁶ are H, or form a substituted or unsubstituted benzo moiety. Preferably R⁵ and R⁶, taken in combination, form a fused 6-membered substituted or unsubstituted benzo moiety yielding a quinolinium ring system.

In the benzazole ring Ql, the ring fragment X is optionally -S-, -O-, -NR⁷-, or -CR⁷R⁸-, wherein R⁷ and R⁸ are as defined previously. Typically R⁷ and R⁸ methyls. Preferably, X is O or S, more preferably X is S.

The quaternizing moiety, TAIL, is attached to the nitrogen atom of Q through a carbon atom and contains a total of 1-22 non-hydrogen atoms that are C, O N or S, such that within TAIL each heteroatom is separated from any adjacent heteroatoms by at least two carbon atoms. TAIL is composed of bonds that are selected from the group consisting of carbon-carbon bonds (C-C), ether bonds (C-O-C), thioether bonds (C-S-C) or amine bonds (C-NR⁹-C). Any carbon atom in TAIL is optionally further substituted by hydroxy, carboxy, sulfo, amino, or ammonium. Any amine, amino or ammonium in TAIL is optionally substituted by an R⁹ that is a C₂-C₆ alkyl that is optionally further substituted by hydroxy, carboxy, sulfo, amino, amino substituted by 1-2 C₁-C₆ alkyls or ammonium substituted by 1-3 C₁-C₆ alkyls. Alternatively, the nitrogen atoms of TAIL form either one or two saturated 5- or 6-membered rings in combination with other C or N atoms in TAIL, such that the resulting rings are pyrrolidines, piperidines, piperazines or morpholines.

In one embodiment, the TAIL moiety includes at least one dialkylamino or a trialkylammonium substituent, where the alkyl substituents are methyl or ethyl. Preferably, TAIL is -CH₃ or CH₂CH₃, or is a C₃-C₂₂ alkyl that is optionally substituted one or more times by hydroxy, carboxy, sulfo, amino, amino substituted by 1-2 C₁-C₆ alkyls, or ammonium substituted by 1-3 C₁-C₆ alkyls. By "sulfo" is meant sulfonic acid (-SO₃H) or the common alkali metal salts of sulfonic acid. More preferably, TAIL is a C₃-C₁₂ alkyl that is linear and saturated, and substituted at its free terminus by hydroxy, carboxy, sulfo, amino, amino substituted by 1-2 C₁-C₆ alkyls, or ammonium substituted by 1-3 C₁-C₆ alkyls. Yet more preferably, TAIL is a C₃-C₄ alkyl that is substituted once by sulfo or carboxy.

The moiety M has the formula 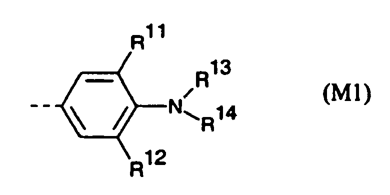 or 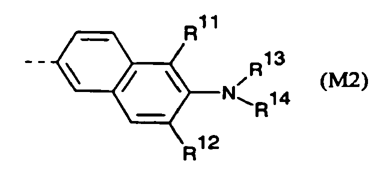 where R¹¹ and R¹² are independently H, F, Cl, or -CH₃; typically H. R¹³ and R¹⁴ are independently C₁-C₁₈ alkyls that are linear, branched, saturated or unsaturated, and are optionally substituted one or more times by F, hydroxy or C₁-C₆ alkoxy. Alternatively, R¹³ and R¹⁴, when taken in combination, form a 5- or 6-membered saturated ring that optionally contains an oxygen heteroatom. In another embodiment of the dyes, R¹¹ taken in combination with R¹³ and R¹⁰ taken in combination with R¹² are independently -(CH₂)₂- or -(CH₂)₃-, forming 5- or 6-membered rings. Preferably R¹³ and R¹⁴ are each linear alkyls, which may be the same or different, each having 4-8 carbon atoms, more preferably each having 5-7 carbon atoms.

Alternatively, M has a formula as depicted in Table I, where R is H, phenyl, sulfophenyl, C₁-C₆ alkyl or C₁-C₆ alkyl substituted by carboxy. Such dyes have been described, for example, by Brooker et al., J. AM. CHEM. SOC. 73, 5326 (1951) (incorporated by reference).

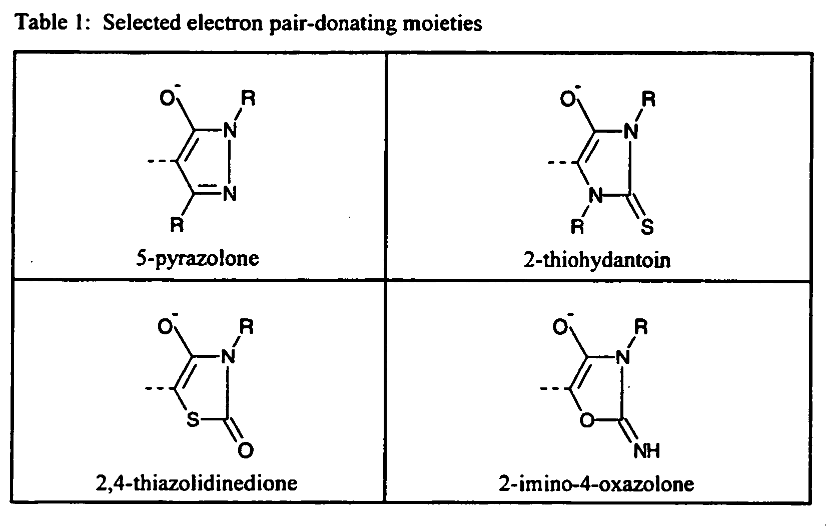

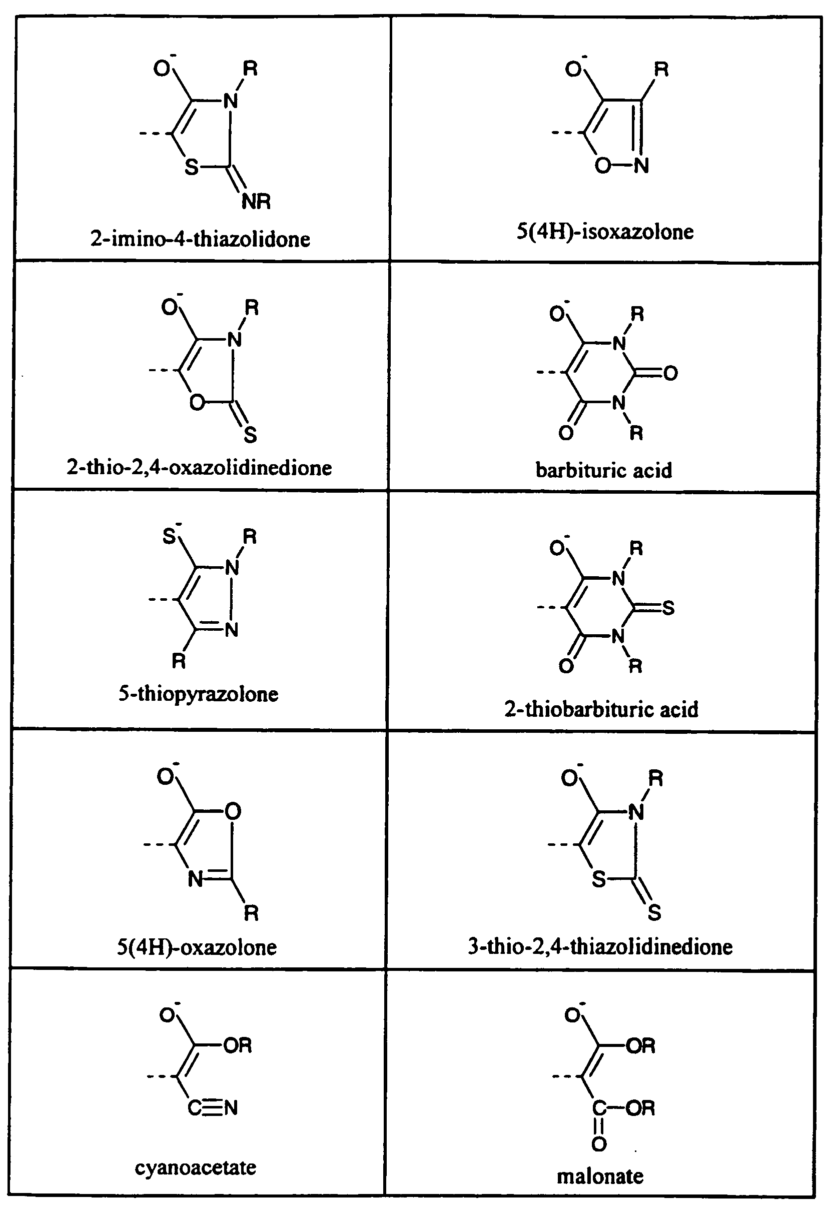

For all dyes, any net positive or negative charges possessed by the dye are balanced by counterion(s), Ψ. Any counterion currently used in conjunction with biomolecules is suitable. Preferred counterions include chloride, iodide, perchlorate, various sulfonates, alkali metal ions, alkaline earth metal ions, transition metal ions, ammonium or substituted ammonium ions.

**Table 2: Selected dyes useful for the present invention**

| Dye no. | Dye Structure |
|---|---|
| 101 | 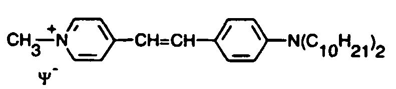 |
| 102 | 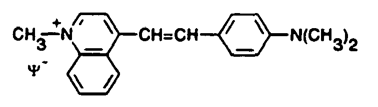 |
| 103 | 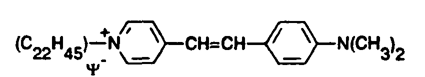 |
| 104 | 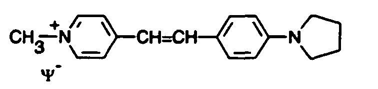 |
| 201 | 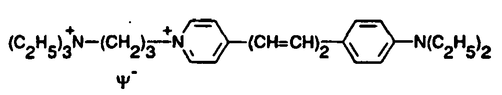 |
| 301 | 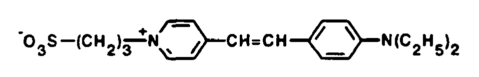 |
| 302 | 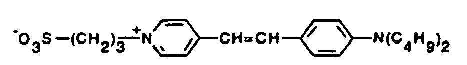 |
| 303 | 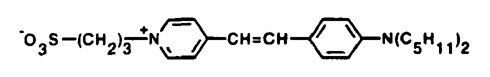 |
| 304 | 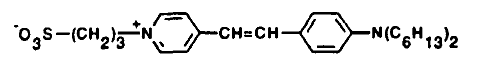 |
| 305 | 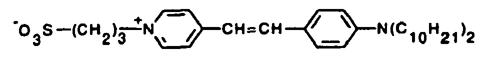 |
| 306 | 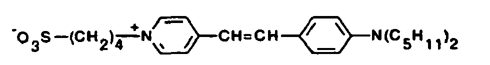 |
| 307 | 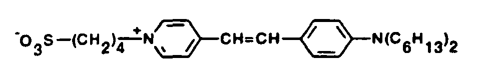 |
| 308 | 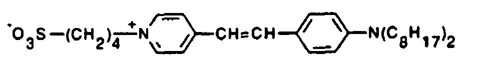 |
| 309 | 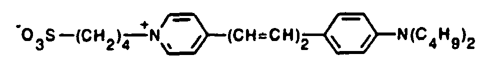 |
| 310 | 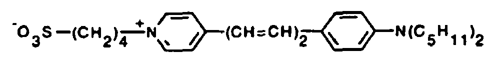 |
| 311 | 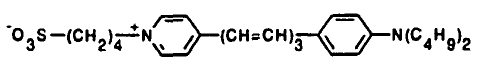 |
| 312 | 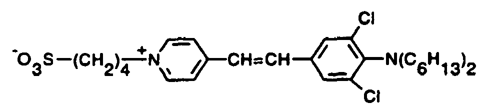 |
| 313 | 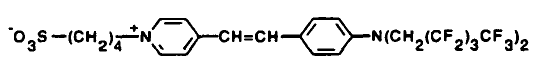 |
| 401 | 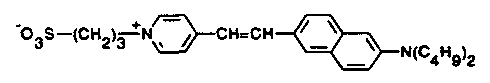 |
| 402 | 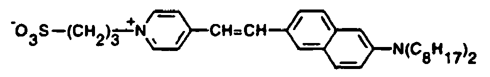 |
| 501 | 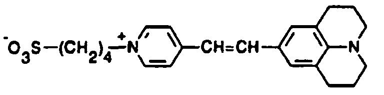 |
| 601 | 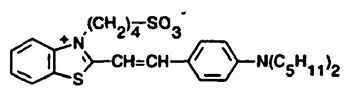 |
| 701 | 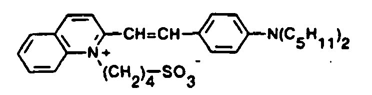 |
| 702 | 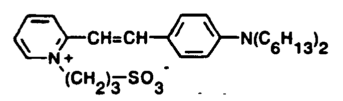 |
| 801 | 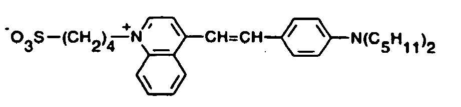 |
| 802 | 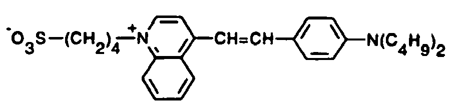 |
| 803 | 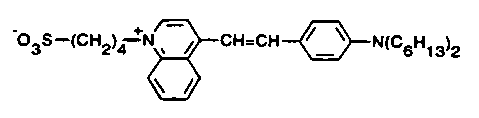 |
| 804 | 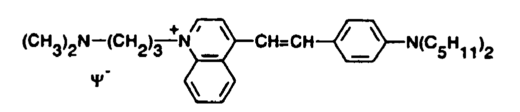 |
| 805 | 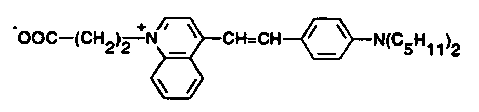 |
| 806 | 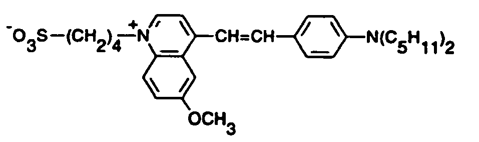 |
| 807 | 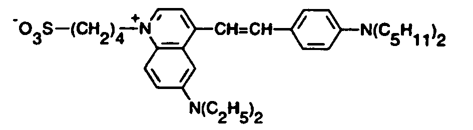 |
| 808 | 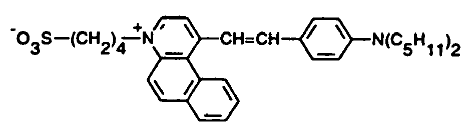 |
| 809 | 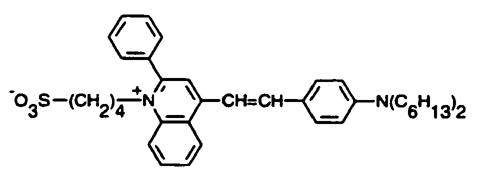 |
| 901 | 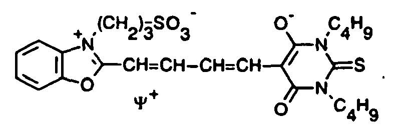 |
| 902 | 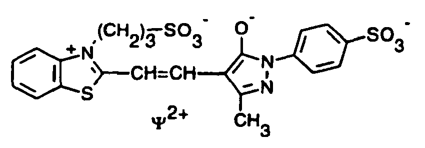 |
| 903 | 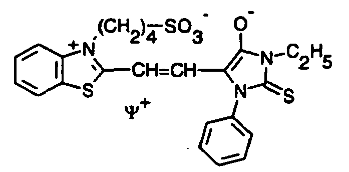 |
| 904 | 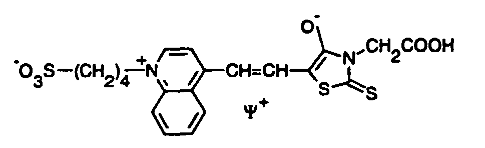 |
| 905 | 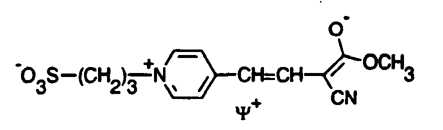 |
| 906 | 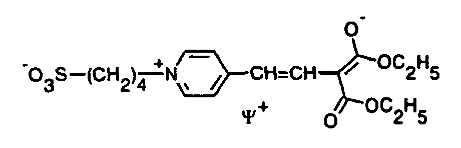 |

The synthesis of merocyanine dyes, including styryl dyes, is well documented. For example, the preparation of selected styryl dyes (RH dyes) is described by Rina Hildesheim (Grinvald et al., BIOPHYS. J. 39, 301 (1982), incorporated by reference), Leslie Loew (Loew et al., J. ORG. CHEM. 49, 2546 (1984), incorporated by reference) and others. Other merocyanine dyes are described by Brooker et al. (J. AM. CHEM. SOC. 73, 5326 (1951), incorporated by reference). Many dyes useful for the invention are available from Molecular Probes, Inc. (Eugene,

The quaternizing moiety ("TAIL") is typically obtained by quaternization of a methyl-substituted pyridine, quinoline or other nitrogen heterocycle by an alkylating agent that already contains other side chain substituents, or that is further reacted to yield the side chain substituents as described by Hildesheim (Grinvald et al. *supra*). In particular, quaternization with propane sultone or butane sultone is convenient and yields preferred dyes. Other substituents are usually added either before or after synthesis of the initial merocyanine dye via an intermediate haloalkyl quaternized heterocycle. However, addition of TAIL is also achieved subsequent to initial dye synthesis, which usually requires an azastilbene or fused analog thereof, as described by Loew et al. (*supra*).

The styryl dyes of the present method are typically prepared by conversion of an aniline derivative that contains the desired R¹, R², R³, and R⁴ substituents to a benzaldehyde, cinnamaldehyde or pentadienal derivative using methods well known in the art. The aldehyde derivative is then condensed with a quaternized pyridinium, quinolinium or benzazolium salt to give the useful dye.

### Method of Use

A staining mixture comprising one or more of the merocyanine dyes described above is combined with a sample mixture that is thought to contain poly(amino acids) for a sufficient time to form a dye-poly(amino acid) complex that gives a detectable colorimetric or fluorescent optical response upon illumination. The complex contains detergent molecules, as described above. The merocyanine dyes present in the complex interact non-covalently with either the poly(amino acid) itself, or with said detergent molecules present in the complex. The sample mixture or the combined mixture is optionally heated, typically to > 90 C. Additional steps are optionally and independently used, in any combination, to provide for separation or purification of the poly(amino acids), for enhancing the detection of the poly(amino acids), or for quantification of the poly(amino acids).

### Sample Mixture

The sample mixture is an aqueous or mostly aqueous solution, or is a solid, paste, emulsion or other solution that is combined with a mostly aqueous solution in the course of labeling. The sample mixture contains or is suspected to contain poly(amino acids). Where the sample mixture is an aqueous solution, the concentration of poly(amino acids) is typically 10 ng/mL - 50 µg/mL, more preferably 50 ng/mL - 5 µg/mL. For electrophoretic gels, the concentration of poly(amino acids) is typically 1 ng/band - 4 µg/band.

Poly(amino acids) suitable for the invention include both synthetic and naturally occurring poly(amino acids). The poly(amino acids) optionally incorporate non-peptide regions including lipid, phosphate, and/or carbohydrate regions. The poly(amino acids) are optionally relatively homogeneous or heterogeneous mixtures of poly(amino acids), or are multi-subunit complexes. The poly(amino acids) in the sample mixture are optionally covalently or non-covalently bound to a solid surface such as glass, plastic, or semiconductor material; or are unbound.

The poly(amino acids) are optionally unmodified, or have been treated with a reagent so as to enhance or decrease the mobility of the poly(amino acid) in an electrophoretic gel, such as by complexing with the peptide (to decrease migration), by cleaving selected peptide bonds (to increase migration of the resulting fragments), by changing the relative charge on the protein (as by phosphorylation or dephosphorylation) or by covalent coupling of a constituent such as occurs during glycosylation. Such interactions are detected by the change in electrophoretic mobility of treated poly(amino acids), relative to untreated poly(amino acids).

Individual amino acids have been stained with these dyes, but typically poly(amino acids) are peptides of mw > 500 daltons (more typically > 800 daltons) or are proteins (Tables 3 and 5). Large poly(amino acids) (> 200,000 daltons)are not well resolved in gels. Smaller poly(amino acids) (< 1000 daltons) are difficult to detect on gels and filters, but are readily detected in solution. In one embodiment of the invention, the poly(amino acids) present are a mixture of poly(amino acids) of different molecular weights (e.g. molecular weight standards).

The sample mixture optionally contains discrete biological ingredients other than the desired poly(amino acids), including other poly(amino acids), amino acids, nucleic acids, carbohydrates, and lipids, which may or may not be removed in this method. In one embodiment, the poly(amino acids) are separated from each other or from other ingredients in the sample by mobility, by size or by binding affinity in the course of the method. Typically intact or fragmented biological membranes, liposomes or detergent micelles in the sample mixture are removed, destroyed or dispersed below the concentration at which they assemble into micelles (critical micelle concentration or CMC) prior to or in the course of labeling with this method. Typically, the sample mixture is essentially cell-free.

### Staining mixture

The staining mixture is combined with the sample mixture so as to facilitate contact between any dye and any poly(amino acids) present in the combined mixture. The staining mixture is typically prepared by dissolving the dye in an organic solvent, such as DMSO, DMF or methanol; and then diluted with an aqueous solution according to the assay being performed. For solution assays, the dye is diluted into an aqueous solution, preferably buffered, that optionally contains a detergent. For staining on gels or membranes, the dyes are diluted into water or buffer, optionally including acetic acid (typically 5%-7%).

For visible color detection, dye concentrations in the staining mixture are typically between 1 µM and 100 µM, preferably between about 5 µM and about 20 µM; more preferably at least 10-15 µM or higher. For fluorescence detection, dye concentrations are typically greater than 0.10 µM and less than 10 µM; preferably greater than about 0.50 µM and less than or equal to about 5 µM; more preferably 1-3 µM. Concentrations below and above these values result in detectable staining for certain poly(amino acids), depending on the sensitivity of the detection method.

A particular dye is generally selected using one or more of the following criteria: sensitivity to poly(amino acids), dynamic range, photostability, staining time, and insensitivity to the presence of nucleic acids. The sensitivity and dynamic range of the dyes is determined using the procedures of Examples 1 and 7. Preferred dyes have a sensitivity of 1-2 ng or less of poly(amino acid) per band in electrophoretic gels, or 10-30 ng or less of poly(amino acid) per mL of solution. Preferably, the dyes have a dynamic range of about 3 or more orders of magnitude of poly(amino acid) concentration for solution assays. Preferred dyes, in an aqueous solution in the absence of poly(amino acids), possess a quantum yield < 0.05, more preferably < 0.01. Preferably, when combined with poly(amino acids), the dyes exhibit a fluorescence enhancement that is > 100-fold, more preferably > 300-fold relative to the dyes in the absence of poly(amino acids). Preferred dyes, when combined with poly(amino acids), have an absorption maximum within 10 nm of 488 nm, 514 nm, 543 nm, or 633 nm.

The invention requires a detergent that is added simultaneously with or as part of the sample mixture or the staining mixture, or is added to the combined mixture as described below. Preferably, the detergent is combined with the sample mixture before the staining mixture is added. The detergent is any amphiphilic surface active agent or surfactant that serves to coat the poly(amino acids), i.e. non-covalently associate with the poly(amino acid). Useful detergents include non-ionic, cationic, anionic, amphoteric and fluorinated surfactants; many are commercially available. Any detergent utilized in protein gel electrophoresis is preferred. Typically, the detergent is an anionic detergent, preferably an alkyl sulfate or alkyl sulfonate salt (typically 6-18 carbons); more preferably, sodium dodecyl sulfate (SDS), sodium octadecyl sulfate, or sodium decyl sulfate; most preferably, SDS.

Preferably detergent in the sample mixture, staining mixture, or combined mixture is present below the CMC for that detergent, in order to avoid poly(amino acid)-free micelle formation. The CMC is a function of the detergent being used and the ionic strength of the solution. For SDS solutions at moderate ionic strength, the CMC is about 0.1 % of the solution by weight. Typically, for prestaining the sample mixture before electrophoretic separation of denatured poly(amino acids) using SDS, the concentration of the detergent is about 1-5% by weight, more typically about 2%. Where the combined mixture is used for a solution assay, the concentration of SDS in the combined mixture is typically less than 1% by weight, preferably 0.05-0.1% by weight. For non-ionic detergents such as PLURONIC and TRITON, the concentration of detergent in the combined mixture is preferably less than about 0.05% by weight.

### Combined Mixture

In one aspect of the invention, the combined mixture is a solution (e.g. Example 7; Table 3); typically, an aqueous solution (preferably buffered) that consists essentially of poly(amino acids), one or more merocyanine dyes, particularly preferred dyes, and a detergent. The aqueous solution is optionally used as a separation medium, such as within a sedimentation gradient (e.g. a sucrose gradient) or when performing capillary electrophoresis. The sample mixture or combined mixture is optionally heated and cooled before the detection or quantification of poly(amino acids).

**Table 3: Properties of selected dyes in solution assays**

| Dye No. | Absorbance (nm)¹ | | Fluorescence (nm)² dye + protein | | Fluorescence Enhancement³ | Dynamic Range Limits⁴ (µg protein) | |
|---|---|---|---|---|---|---|---|
| | Free Dye | Dye+ Protein | Excitation | Emission | | Lower | Upper |
| 101 | 448 | 447 | 469 | 583 | 1.1 | 10 | 10 |
| 102 | 509 | 508 | 526 | 624 | 2 | 0.05 | 5 |
| 104 | 463 | 468 | 469 | 570 | 12.7 | 0.05 | 5 |
| 106 | 440 | 437 | 448 | 578 | 1.1 | 50 | 50 |
| 201 | 480 | 507 | 494 | 635 | 142 | 0.05 | 10 |
| 301 | 473 | 474 | 473 | 576 | 23.5 | 0.5 | 25 |
| 302 | 479 | 476 | 469 | 576 | 188 | 0.05 | 5 |
| 303 | 475 | 476 | 472 | 569 | 363 | 0.05 | 5 |
| 304 | 464 | 473 | 470 | 569 | 533 | 0.05 | 5 |
| 305 | 465 | 465 | 471 | 568 | 12.3 | 0.5 | 0.5 |
| 306 | 472 | 472 | 469 | 567 | 307 | 0.05 | 5 |
| 307 | 461 | 472 | 469 | 569 | 443 | 0.05 | 5 |
| 308 | 458 | 470 | 470 | 568 | 202 | 0.05 | 5 |
| 309 | 507 | 507 | 483 | 629 | 283 | 0.05 | 5 |
| 310 | 507 | 507 | 483 | 628 | 356 | 0.05 | 5 |
| 311 | 479 | 508 | 504 | 669 | 158 | 0.25 | 5 |
| 401 | 472 | 476 | 470 | 611 | 679 | 0.25 | 5 |
| 402 | 455 | 467 | 472 | 597 | 20.2 | 0.05 | 1 |
| 501 | 506 | 506 | 496 | 589 | 76.2 | 0.25 | 10 |
| 601 | 471 | 472 | 545 | 584 | 154 | 0.05 | 10 |
| 701 | 508 | 513 | 526 | 594 | 534 | 0.25 | 10 |
| 801 | 538 | 543 | 547 | 631 | 1285 | 0.25 | 10 |
| 802 | 542 | 543 | 554 | 623 | 351 | 0.05 | 10 |
| 803 | 507 | 537 | 544 | 630 | 2238 | 0.25 | 10 |
| 901 | 506 | 506 | 560 | 570 | 66.2 | 2.5 | 50 |

| | | | | | | | |
|---|---|---|---|---|---|---|---|
| ¹ Absorbance maxima are determined using 1 µM dye + 0.05% SDS +/- 150 µg/mL BSA (bovine serum albumin) in a 2.0 mL volume. ² Fluorescence excitation and emission spectra are measured using 1 µM dye + 150 µg/mL BSA + 0.05% SDS in 2.0 mL volume. ³ Fluorescence enhancements are calculated using I µM dye +/- 500 µg/mL BSA in 2.0 mL at fluorescence excitation and emission maxima. ⁴ Dynamic range limits are determined by titrating 1 µM dye with 0.05 to 50 µg protein in a 96-well microplate in a 200 µL volume. Three protein solutions are titrated separately for each dye: lysozyme, BSA and a protein mixture that contains equal weight concentrations of IgG, avidin, streptavidin, cellulase and ovalbumin. The lower limit of detection is the amount of protein required to produce fluorescence 10% above background. The upper limit is where the linearity of signal with respect to protein concentration starts to degrade for the protein type producing signal saturation at the lowest concentration. | | | | | | | |

In another aspect of the invention, the merocyanine dyes are optionally used to prestain poly(amino acids) prior to separation; or are present as a component of the mobile phase during separation (e.g. Example 12) in gel or capillary electrophoresis. Alternatively, separated poly(amino acids) in electrophoretic (denaturing or non-denaturing) gels are post-stained using the staining mixture, or are transferred to a filter membrane or blot or other solid or semi-solid matrix before being combined with the staining mixture (e.g. Examples 1, 2, 3, and 5). Typically, polyacrylamide or agarose gels are used for electrophoresis. Alternatively, the gel is an iso-electric focusing gel, a gradient gel, a two-dimensional gel, or the gel is used for gel-mobility-shift analysis. The sample mixture or combined mixture is optionally heated before being applied to denaturing gels. The sensitivities of selected dyes of the present invention when used to post-stain electrophoretic gels are listed in Table 4.

**Table 4: Labeling sensitivity in gels of selected dyes¹**

| Dye No. | Color ² | Sensitivity ³ (ng/band) | Background Staining |
|---|---|---|---|
| Coomassie Brilliant Blue | blue* | 30-60 (low) | very high: requires destaining |
| Nile red | red/orange* | 30-60 (low) | low/med |
| Silver staining | tan to black* | variable 2-10 (high) | varies, low to very high even with destaining |
| 101 | faint greenish | low | med |
| 102 | light orange | med | med |
| 103 | red/orange | low | low/med |
| 104 | yellow | med | low/med |
| 201 | red/orange | med | low/med |
| 301 | barely visible | low | low |
| 302 | greenish/pink | low | low |
| 303 | pink/yellow to orange | med | med |
| 304 | bright orange | high | low/med |
| 305 | light orange | low | med |
| 306 | orange | med | low |
| 307 | bright orange | high | low/med |
| 308 | orange | high | med |
| 309 | pink/orange | med | low |
| 310 | red/orange | high | low/med |
| 311 | light orange | low | low/med |
| 401 | orange | med | med |
| 402 | red/orange | high | low |
| 501 | orange | med | low/med |
| 601 | red/orange | high | low |
| 701 | red/orange | high | low |
| 801 | red | high | low |
| 803 | red | high | low |
| 802 | red | high | low |
| 901 | orange | med | med/high |

| | | | |
|---|---|---|---|
| ¹ All electrophoretic gels are SDS acrylamide mini-gels (Bio-Rad) ² Color refers to the color of the fluorescence emission of the stained protein bands, except where marked with * where color refers to colorimetric appearance under white light. ³ Sensitivity refers to the lower limit of protein detection, per band, using a 4X dilution series of BSA (using the procedure of Example 1). The sensitivity is defined to be the band containing the smallest amount of protein that is easily detectable by eye in a black and white Polaroid photograph taken at an optimal exposure. "Low" sensitivity means a detection limit comparable to that found with CBB and nile red; "high" sensitivity means a detection limit comparable to that found with silver staining; "medium" sensitivity is intermediate between the two ranges. | | | |

The sensitivity of the present method for a variety of poly(amino acids) was compared to that of silver staining and CBB staining molecular weights (Table 5).

**Table 5: Sensitivity of detection as a function of poly(amino acid) composition**

| Protein | Molecular Weight (daltons) | Sensitivity Dye 304 (ng) | Sensitivity Silver Staining (ng) | Sensitivity CBB Staining (ng) |
|---|---|---|---|---|
| myosin | 200,000 | 1 | 1 | 8-16 |
| β-galactosidase | 116,250 | 1 | 2-4 | 8-16 |
| phosphorylase B | 97,400 | 2-4 | 1 | 30-60 |
| fructose-6-phosphate kinase | 85,200 | 1-2 | 2-4 | 31-62 |
| bovine serum albumin | 66,200 | 2-4 | 2-4 | 16-30 |
| bovine cytochrome C oxidase (COX) subunit I | 56,900 | 1-2 | 26-52 | 105 |
| glutamate dehydrogenase | 55,400 | 1-2 | 2-4 | 16 |
| IgG (heavy chain) | 50,000 | 2 | 4 | 34-69 |
| ovalbumin | 45,000 | 1-2 | 1-2 | 16-30 |
| Protein A | 41,000 | 1 | 1-2 | 16 |
| aldolase | 39,200 | 1-2 | 1 | 31-62 |
| carbonic anhydrase | 31,000 | 2 | 4-8 | 16-30 |
| COX subunit II | 29,900 | 1-2 | 2-4 | 27-54 |
| triose phosphate isomerase | 26,600 | 1 | 1 | 16-31 |
| COX subunit III | 26,000 | 0.8 | 0.8-1.5 | 48 |
| Protein G | 26,000 | 2 | 16 | 32-64 |
| IgG (light chain) | 25,000 | 14 | 56 | 166 |
| soybean trypsin inhibitor | 21,500 | 1-2 | 2-4 | 16-30 |
| histone H1 | 20,000 | 10 | 80 | 80-160 |
| β-bungarotoxin subunit | 20,000 | 1.25-2.5 | 1.25 | 41 |
| COX subunit IV | 17,100 | 0.5-1 | 4-8 | 16-32 |
| avidin | 4 x 16,500 | 2 | 2-4 | 16 |
| streptavidin | 4 x 15,000 | 2 | 16 | 32-64 |
| histone H3 | 15,700 | 7.5 | 15 | 30-60 |
| NEUTRALITE avidin | 15,000 | 1-2 | 62 | 62 |
| lysozyme | 14,400 | 1-2 | 4 | 16-30 |
| histone H2A/H2B | 13,700 | 7 | 14-27 | 54 |
| pancreatic RNase A | 13,700 | 4-8 | 16-30 | 125 |
| COX subunit Va | 12,400 | 1 | 45 | 16-32 |
| histone H4 | 11,200 | 3 | 22-45 | 45 |
| COX subunit Vb | 10,700 | 1 | 4-8 | 16-32 |
| COX subunit VIa | 9,400 | 1 | 4-8 | 16-32 |
| α-bungarotoxin | 8,000 | 4-8 | 2-4 | 31-62 |
| β-bungarotoxin subunit | 7,000 | 3-6 | --- | 175-350 |
| aprotinin | 6,500 | 2 | 4-8 | 16-30 |
| human insulin | 6,000 | 8 | 8 | 125-250 |

Using the present invention, destaining of stained gels is generally not necessary for either colorimetric or fluorescent detection. At very high dye concentrations, destaining is optionally used to improve visible color detection in gels. Gels are optionally washed briefly after staining to prevent transfer of dye to other surfaces.

### Additional reagents

The method of the present invention optionally further comprises addition of an additional reagent, simultaneously or sequentially, to the sample mixture, the staining mixture, or the combined mixture. The additional reagent incorporates a means for producing a detectable response, including the change in, or appearance of, color, fluorescence, reflectance, pH, chemiluminescence, infrared spectra, magnetic properties, radioactivity, light scattering, x-ray scattering, or production of an electron-rich substrate. An additional reagent is optionally a detection reagent that colocalizes with poly(amino acids) in general or with specific poly(amino acids); or is a probe for a specific component of the sample mixture (e.g. a nucleic acid stain; a detectable member of a specific binding pair); or changes the mobility of the poly(amino acid) as described above. In one embodiment of the invention, the additional reagent is one or more additional merocyanine dyes, including preferred embodiments described above. Where the additional merocyanine dye(s) have overlapping spectral characteristics such that energy transfer occurs, the labeled poly(amino acids) exhibit an extended Stokes shift. Alternatively, the additional reagent is another protein stain (such as CBB or silver stain) such that labeling of the poly(amino acids) is enhanced by the colocalization of staining.

### Illumination and Observation

The combined mixture of dye and sample is illuminated to give an optical response that is an absorption of visible light (colorimetric response), or is a fluorescence emission (fluorescence response), or both. Illumination is by a light source capable of exciting the dye-poly(amino acid) complex, typically at or near the wavelength of maximum absorption of the dye-poly(amino acid) complex, such as an ultraviolet (254-370 nm) or visible (490-550 nm) wavelength emission lamp, an arc lamp, a laser, or even sunlight or ordinary room light. Preferably, the sample is excited with a wavelength within 20 nm of the maximum absorption of the dye-poly(amino acid) complex.

Preferably, the dye-poly(amino acid) complexes possess an absorption maximum between 480 and 650 nm, more preferably between 488 and 550 nm, most preferably matching the wavelength of a laser illumination source. The complexes also preferably excite in the UV at or near 300 nm.

The optical response is detected qualitatively, or optionally quantitatively, by means that include visual inspection, CCD cameras, video cameras, photographic film, or the use of instrumentation such as laser scanning devices, fluorometers, photodiodes, quantum counters, epifluorescence microscopes, scanning microscopes, flow cytometers, fluorescence microplate readers, or by means for amplifying the signal such as photomultiplier tubes. Recording the optical response using POLAROID film results in enhanced sensitivity of signal.

Quantification of the poly(amino acid) is typically performed by comparison of the optical response to a prepared standard or to a calibration curve, obtained from a standard dilution of a known concentration of a poly(amino acid) or poly(amino acid) mixture. Alternatively, the standard curve is generated by comparison with a reference dye or dyed particle that has been standardized versus the target dye-poly(amino acid) complex.

Due to the simplicity of use of the instant dyes, said dyes are particularly useful in the formulation of a kit for the labeling of poly(amino acids), comprising one or more merocyanine dyes (preferably in a stock solution) and instructions for the use of the dye, optionally including a reference standard. In one embodiment, the instructions specify that the sample mixture and staining mixtures are applied, either simultaneously or sequentially to a solid or semi-solid support, the resulting color or fluorescence of which is then compared to a calibrated standard to determine the presence, and optionally the concentration, of poly(amino acid) present in the sample mixture. The support, dye solution, calibration standard and instructions for use, in combination, comprise a "dipstick" protein assay kit, allowing the rapid and convenient determination of protein concentration.

The examples below are given so as to illustrate the practice of this invention. They are not intended to limit or define the entire scope of this invention.

### EXAMPLES

### Example 1. Detection of proteins in SDS-polyacrylamide gels:

The pure protein or mixture of proteins is prepared in Loading Buffer (50 mM Tris-HCl, pH 6.8, 2% SDS, 10% glycerol, and 0.015% bromophenol blue). Dithiothreitol is added to each sample, to 0.1 M. The samples are heated 4-5 minutes at 90-95 °C and loaded onto a 15% Tris-glycine polyacrylamide mini- or full-sized gel containing 0.05%-0.1% SDS, with a 4% stacking gel. The gel is electrophoresed under standard conditions, in Tris-glycine buffer containing 0.05%-0.1% SDS. The resulting gel is transferred to a staining dish containing 1-3 µM Dye 304 in 7.5% acetic acid (in water). The staining solution is gently agitated for 45-60 minutes. After 10 minutes, the protein bands are readily apparent, but sensitivity improves over about 30-40 minutes. The gel is removed from the staining dish, rinsed briefly in 7.5% acetic acid and transferred to a UV-transilluminator. The gel is photographed using 300 nm transillumination and black and white POLAROID 667 print film with a Wratten 9 gelatin filter, or using a CCD camera with UV illumination near 300 nm. The stained bands exhibit bright orange fluorescence. Under normal room lights, the stained bands appear as orange bands. Other dyes of the invention (See Table 3) yield stained gels that possess bands having visible coloring from yellow to purple and fluorescence emission from green to red.

### Example 2. Detection of proteins in non-denaturing polyacrylamide gels:

A protein dilution series is prepared in Native Gel Loading Buffer (125 mM Tris-HCl, pH 6.8, 10% glycerol and 0.015% bromophenol blue). The samples are loaded onto a Tris-HCl non-denaturing polyacrylamide gel, and the gel is electrophoresed under standard conditions. The electrophoresed gel is stained and photographed as in Example 1. Staining sensitivity under these conditions is typically somewhat protein-selective.

### Example 3. Detection of peptides in Tris-Tricine gels:

A dilution series of a short peptide is prepared in Loading Buffer. To each sample is added dithiothreitol to 0.1 M, the samples are heated 4-5 minutes at 90-95 °C then loaded onto 16.5% Tris-tricine gel. The gel is electrophoresed under standard conditions. The gel is stained and the protein bands are visualized as in Example 1. Polypeptides as small as 20 amino acids are readily detected using this method. Peptides such as a tetramer of the heptapeptide repeat found at the C-terminus of eukaryotic RNA polymerase II, tryptic peptides of trypsin and the small subunit of β-bungarotoxin, which are not stained with either CBB or silver staining, are readily detectable.

### Example 4. Detection of proteins in 2-dimensional gels:

A mouse cytosolic extract (i.e., a whole cell lysate) is prepared and separated on a 2-dimensional gel under standard conditions. The gel is stained and visualized as in Example 1. An identical gel is stained in parallel with silver using a commercially available kit (Bio-Rad). The gel stained using the present invention exhibits more protein spots than the silver stained gel, indicating that the present invention is less protein-selective than silver staining. The signal intensity obtained with the instant method is more directly proportional to protein concentration than that obtained with silver staining. The sensitivity of staining using the present invention is superior to that of silver staining for detection of low molecular weight proteins (Table 3).

### Example 5. Detection of proteins on filter membranes:

The protein is diluted in TBS (20 mM Tris-HCl, pH 7.5, 500 mM NaCl), then applied to a nylon or nitrocellulose filter membrane. The membrane is washed with TBS, then incubated with 2 µM Dye 803 in 7.5% acetic acid or TBS. Alternatively, the proteins are first separated by gel electrophoresis and transferred to a nylon or nitrocellulose filter membrane using standard procedures. The blot is stained as above. The blot is illuminated at 300-365 nm. Protein spots or bands are fluorescent. Blots are photographed as in Example 1. Stained proteins appear as faint white spots or white bands. Staining under these conditions is somewhat protein selective. Proteins are optionally detected using laser illumination sources.

### Example 6. Western blotting of stained proteins:

A dilution series of bovine heart cytochrome oxidase complex (COX) is prepared in water. Samples are loaded onto a 12% polyacrylamide gel and the gel is electrophoresed under standard conditions. The resulting gel is cut in half, and one half is stained with Dye 801 in transfer buffer (20% MeOH, 25 mM Tris-glycine, pH 8.3, 192 mM glycine) and visualized as in Example 1 above. The proteins are electrophoretically transferred from both halves of the gel to a filter membrane, according to standard methods. The blot is blocked, probed with mouse monoclonal antibodies directed against specific COX subunits, and the resulting signals are visualized using nitroblue tetrazolium and 5-bromo-4-chloro-3-indolyl phosphate, using standard procedures. The two halves of the blot show equal sensitivity for detection of the COX subunits, indicating that staining proteins with the invention does not interfere with later immunological detection.

### Example 7. Detection of proteins in solution:

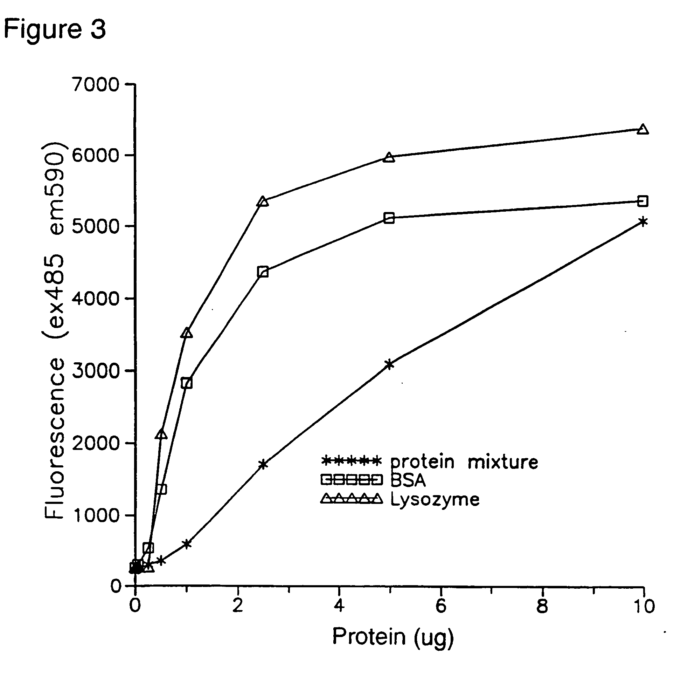

A dilution series of proteins is prepared in 10 mM Tris-HCl, pH 7.5, containing 0.05% SDS. An equal volume of 2 µM Dye 307 is added to each sample. The samples are incubated 15 minutes at room temperature. The fluorescence intensity of each sample is measured using 490 nm excitation, using a fluorometer or fluorescence microplate reader. The fluorescence of Dye 307 in buffer alone (control) is subtracted from that of the protein-containing samples. Protein concentrations are determined by comparing the net fluorescence with that of a standard, such as bovine serum albumin, or protein mixture (Figure 3).

### Example 8. Detection of proteins in gels in the presence of nucleic acids:

An extract from *E. coli* is prepared using standard methods, by suspending a bacterial cell pellet directly in Loading Buffer and heating it as in Example 1. The resulting sample is pipeted up and down repeatedly, through a fine bore syringe, to shear large DNA molecules. The sample is diluted serially and loaded onto a denaturing SDS gel in two duplicate dilution series and electrophoresed, as in Example 1. The electrophoresed gel is cut in half. One half is stained with Dye 304 according to Example 1. The other half is stained with silver according to standard methods. The silver-stained half shows staining of bands that correspond to nucleic acids as well as protein. The half stained with Dye 304 exhibits only those bands that result from protein staining.

### Example 9. Detection of proteins in gels using a laser-excited gel scanner:

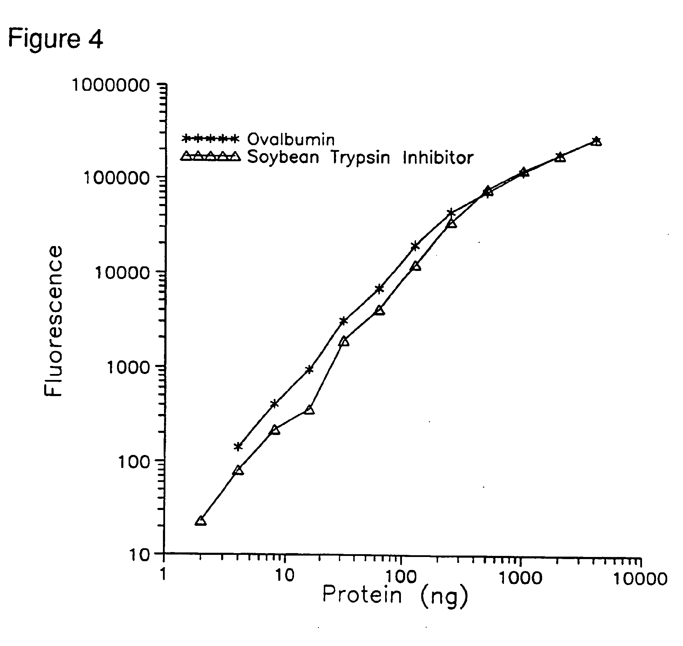

Gels are prepared and stained as in Examples 1, 2, 3 or 4. The stained gels are scanned using a laser-excited gel scanner. Stained proteins display fluorescence emission having a dynamic range of at least 3 orders of magnitude in protein concentration, with a sensitivity of a single ng per protein band (Figure 4, Table 2). Stained gels can also be analyzed using visible light lasers, or other scanners with light sources that overlap with the excitation spectra for the dye used.

### Example 10. Detection of proteins spotted on a TLC plate or filter membrane:

Dilution series of BSA, lysozyme and an equi-mass mixture of myosin, β-galactosidase, phosphorylase B, bovine serum albumin, ovalbumin, carbonic anhydrase, trypsin inhibitor, lysozyme and aprotinin are prepared in 10 mM Tris-HCl, pH 7.5, containing 0.05% SDS. An equal volume of Dye 803 in the same buffer is added to each and the samples are incubated for 15 minutes. A 1-5 µL aliquot of each mixture is spotted onto a thin layer chromatography plate or a nylon or nitrocellulose filter membrane. Protein-containing spots are detected colorimetrically under room light, or are detected by fluorescence following ultraviolet or visible illumination. The color intensity or fluorescence intensity of a given spot is indicative of the amount of protein present in that spot. The amount of protein in an unknown sample is determined by comparing either the colorimetric or fluorescence intensity with that of a standard of known concentration.

### Example 11. Staining protein gels with dye in the running buffer:

Proteins are loaded and run on standard SDS gels, using standard methods, except that the running buffer contains 0.05% SDS and 1-3 µM Dye 801. The stained gels are photographed directly after electrophoresis (as in Example 1) or are destained in 7.5% acetic acid for 20-50 minutes prior to photography. The sensitivity obtained is about the same as obtained by staining gels after electrophoresis. Migration of protein bands can be monitored through the glass plates that support the gel, during electrophoresis.

### Example 12. Prestaining of proteins prior to electrophoresis:

Proteins are diluted in Loading Buffer, heated to 90-95 °C for 4-5 minutes then cooled to room temperature. Dye 304 is added to 10 mM, and the samples are loaded onto a 12% polyacrylamide gel. The gel is electrophoresed and visualized using ultraviolet illumination as in Example 1, or as in Example 9, or with a CCD camera. The sensitivity of this method is somewhat less than in Examples 1 and 11.

It is to be understood that, while the foregoing invention has been described in detail by way of illustration and example, numerous modifications, substitutions, and alterations are possible without departing from the scope of the invention as described in the following claims.

## Claims

1. A method of detecting for a poly(amino acid) in a sample mixture, comprising the steps of:
a) preparing, from the sample mixture and a staining mixture that contains one or more merocyanine dyes, a cell-free combined mixture containing the poly(amino acid) (if present), the one or more merocyanine dyes and a detergent;
b) incubating the combined mixture to allow the dye in the staining mixture to associate with any poly(amino acid) in the sample mixture to form a dye-poly(amino acid) complex that gives a detectable optical response upon illumination;
c) illuminating a product obtained using step (c), e.g. dye-poly(amino acid) complex; and
d) observing the optical response.

2. A method according to Claim 1, wherein between steps a) and b) the sample mixture or combined mixture is heated.

3. A method, as in Claim 1 or 2, wherein the detergent is added simultaneously with or as part of the sample mixture or the staining mixture, or is added after the sample and staining mixtures have been combined.

4. A method, as claimed in Claim 1, 2 or 3, wherein each merocyanine dye independently has the formula
Q-B-M
where Q is 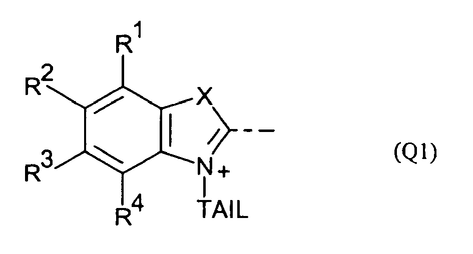 or 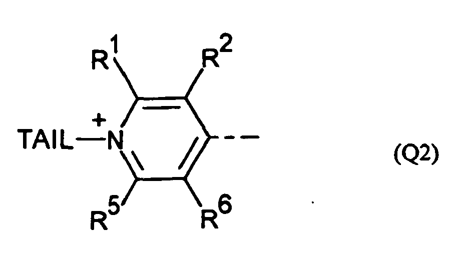 or 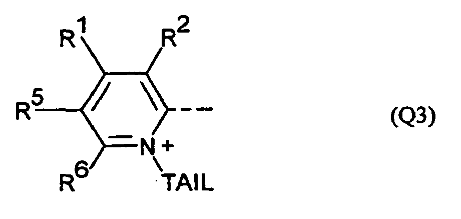 where
R¹, R², R³ and R⁴ are independently H, Cl, F, C₁-C₆ alkyl, C₁-C₆ perfluoroalkyl, C₁-C₆ alkoxy, amino, or amino substituted by 1-2 C₁-C₆ alkyls;
R⁵ and R⁶ are independently H, Cl, F, C₁-C₆ alkyl, C₁-C₆ perfluoroalkyl, C₁-C₆ alkoxy, amino, or amino substituted by 1-2 C₁-C₆ alkyls, or phenyl; or R⁵ and R⁶ taken in combination form a fused 6-membered aromatic ring that is optionally substituted one or more times by substituents which may be the same or different and are selected from Cl, F, C₁-C₆ alkyl, C₁-C₆ perfluoroalkyl, C₁-C₆ alkoxy, amino and amino substituted by 1-2 C₁-C₆ alkyls, or said fused 6-membered aromatic ring is optionally substituted by an additional fused 6-membered aromatic ring that is optionally substituted by substituents which may be the same or different and are selected from Cl, F, C₁-C₆ alkyl, C₁-C₆ perfluoroalkyl, C₁-C₆ alkoxy, amino and amino substituted by 1-2 C₁-C₆ alkyls;
X is -S-, -O-, -NR⁷-, or -CR⁷R⁸-, wherein R⁷ and R⁸ are independently H, C₁-C₆ alkyl or phenyl; or R⁷ and R⁸ taken in combination complete a 5- or 6-membered saturated ring;
TAIL is attached to Q through a carbon atom and contains 1-22 non-hydrogen atoms, that are C, O, N or S, such that each heteroatom is separated from any adjacent heteroatoms by at least two carbon atoms; and further such that TAIL is composed of carbon-carbon bonds (C-C), ether bonds (C-O-C), thioether bonds (C-S-C) or amine bonds (C-NR⁹-C); where any carbon atom in TAIL is optionally further substituted by hydroxy, carboxy, sulfo, amino or ammonium; and where any amine bond, amino or ammonium in TAIL is optionally substituted by an R⁹ that is a C₂-C₆ alkyl that is optionally further substituted by hydroxy, carboy, sulfo, amino, amino substituted by 1-2 C₁-C₆ alkyls, or ammonium substituted by 1-3 C₁-C₆ alkyls, or said N atoms form either one or two saturated 5- or 6-membered rings in combination with additional C or N atoms in TAIL;
B is
-(CR⁷=CR⁸)ₙ-
where R⁷ and R⁸ are as defined previously;
n = 1, 2 or 3;
and M is 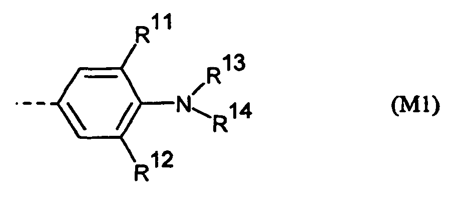 or 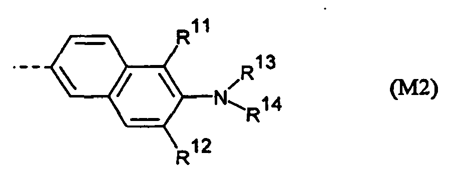 where
R¹¹ and R¹² are independently H, F, Cl, or -CH₃;
R¹³ and R¹⁴ are independently C₁-C₁₈ alkyl that is linear, branched, saturated or unsaturated, and is optionally substituted one or more times by F, hydroxy or C₁-C₆ alkoxy; or R¹³ and R¹⁴ taken in combination form a 5- or 6-membered saturated ring containing 0 or 1 oxygen heteroatoms; or R¹³ taken in combination with R¹¹ and R¹⁴ taken in combination with R¹² independently are -(CH₂)₂- or -(CH₂)₃-;
or M is 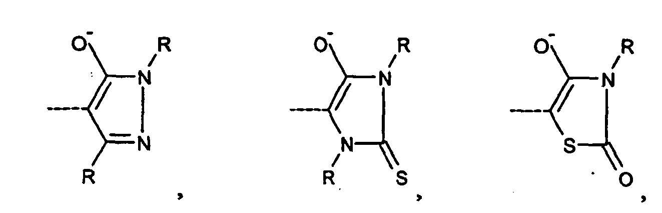 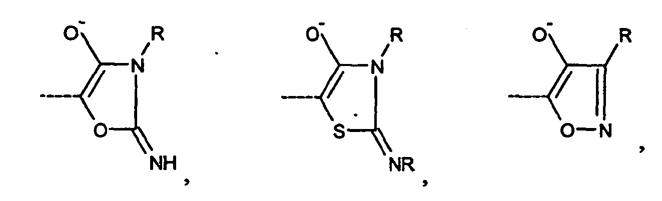 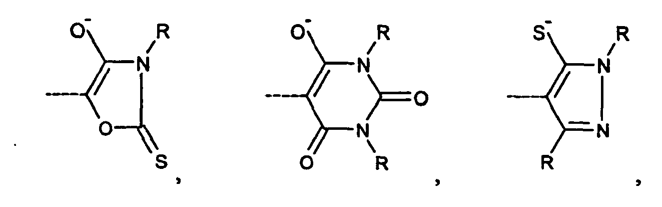 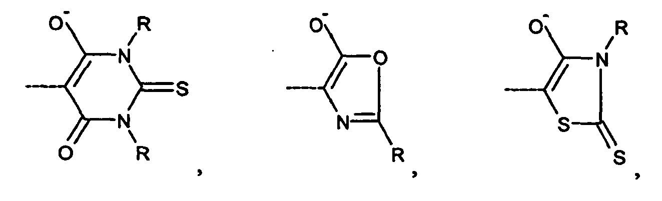 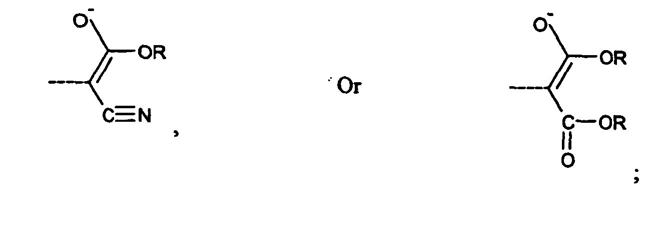 where R is independently H, phenyl, sulfophenyl, C₁-C₆ alkyl, or C₁-C₆ alkyl substituted by carboxy.

5. A method, as in Claim 1 or 2, wherein the staining mixture contains one or more styryl dyes independently having the formula Q-B-M, wherein Q and B are as defined in Claim 4 and M has the formula M1 or M2 as defined in Claim 4.

6. A method, as in Claim 4 or 5, wherein the staining mixture contains a dye wherein:
(i) M has the formula M1; or
(ii) M has the formula M2

7. A method, as in Claim 4 or 5, wherein the staining mixture contains a dye which has the formula

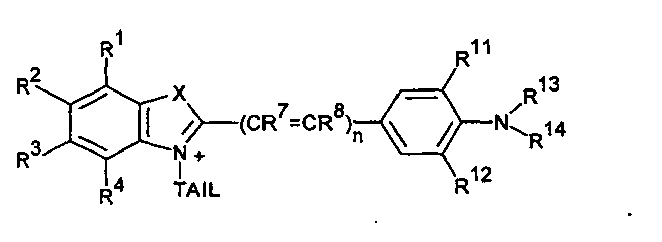

8. A method, as in Claim 4 or 5, wherein the staining mixture contains a dye which has the formula

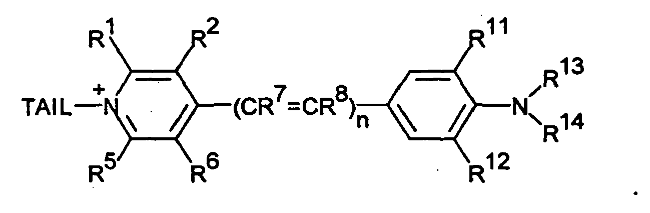

9. A method, as in Claim 4 or 5, wherein the staining mixture contains a dye which has the formula

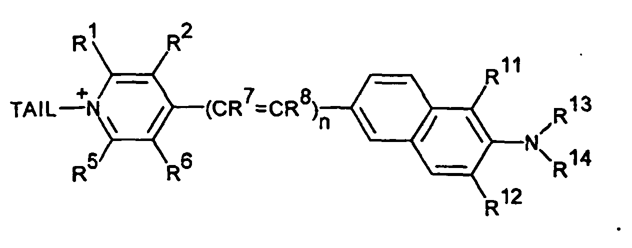

10. A method, as in Claim 4 or 5, wherein the staining mixture contains a dye which has the formula 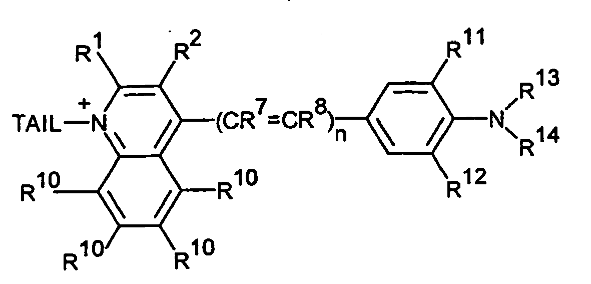 where each R¹⁰ is independently H, Cl, F, C₁-C₆ alkyl, C₁-C₆ perfluoroalkyl, C₁-C₆ alkoxy, amino, amino substituted by 1-2 C₁-C₆ alkyls, or any two adjacent R¹⁰ substituents, when taken in combination, form a fused 6-membered aromatic ring that is optionally substituted by substituents which may be the same or different and are selected from Cl, F, C₁-C₆ alkyl,C₁-C₆ perfluoroalkyl, C₁-C₆ alkoxy, amino and amino substituted by 1-2 C₁-C₆ alkyls.

11. A method, as in any of Claims 4 to 10, wherein the staining mixture contains a dye wherein,
TAIL is -CH₃, or CH₂CH₃; or TAIL is a C₃-C₂₂ alkyl chain that is linear or branched, saturated or unsaturated, that is optionally substituted one or more times by hydroxy, carboxy, sulfo, amino, amino substituted by 1-2 C₁-C₆ alkyls, or ammonium substituted by 1-3 C₁-C₆ alkyls:

12. A method, as in Claim 4 or 5, wherein the staining mixture contains a dye wherein,
M has the formula M1 or M2;
X is O or S;
B is -(CH=CH)ₙ-;
n = 1 or 2;
R¹¹ and R¹² are each H;
R¹³ and R¹⁴ are independently C₄-C₈ alkyl; and
TAIL is sulfopropyl, sulfobutyl, aminopropyl or aminobutyl, the amines of which are substituted 1-3 times by any combination ofH, methyl or ethyl.

13. A method, as in Claim 12, wherein the staining mixture contains a dye wherein,
R¹, R², R³ and R⁴ are each H;
R⁵ and R⁶ are each H; and
said dye is present in the combined mixture at a concentration of 0.10 µM - 10 µM for a fluorescent optical response or 10 µM - 100 µM for colorimetric optical response; and
said poly(amino acid) has a molecular weight of 500-200,000 daltons.

14. A method, as in any of Claims 4 to 6 or 8 to 10 wherein
(i) the staining mixture contains a dye wherein Q is Q1;
(ii) the staining mixture contains a dye wherein Q is Q2; or
(iii) the staining mixture contains a dye wherein Q is Q3.

15. A method, as in any of Claims 1 to 14, further comprising transferring the sample mixture to a solid or semi-solid matrix before or after combining with the staining mixture.

16. A method, as in any of Claims 1 to 15, further comprising removing, destroying, or dispersing below the critical micelle concentration any biological membranes that are present in the sample mixture; and optionally further comprising
(i) quantitating said poly(amino acid) by measuring said detectable optical response and comparing said measurement with a standard; and/or
(ii) electrophoretically separating the sample mixture before, after, or while it is combined with the staining mixture; and/or
(iii) adding an additional reagent to the sample mixture, the staining mixture, or the combined mixture.

17. A method, as in any of Claims 1 to 15, further comprising heating the sample mixture or combined mixture; and optionally further comprising
(i) quantitating said poly(amino acid) by measuring said detectable optical response and comparing said measurement with a standard; and/or
(ii) electrophoretically separating the sample mixture before, after, or while it is combined with the staining mixture; and/or
(iii) adding an additional reagent to the sample mixture, the staining mixture, or the combined mixture.

18. A method, as in any of Claims 1 to 17, wherein said detectable optical response is a fluorescence response.

19. A method, as in any of Claims 1 to 18, wherein the sample mixture contains a poly(amino acid) and step (c) is illumination of a complex formed by the poly(amino acid) and the dye in step (b), step (d) being observation of the optical response of the complex.

20. A combination comprising:
a) one or more styryl dyes having the formula
Q-B-M
where Q is Q1, Q2 or Q3 as defined in Claim 4;
B is
-(CR⁷=CR⁸)ₙ-
where R⁷ and R⁸ are as defined in claim 1
n = 1, 2 or 3;
and M is M1 or M2 as defined in Claim 4;
b) a detergent; and
c) a poly(amino acid),
in a cell-free aqueous solution where said detergent is present at a concentration less than the critical micelle concentration for that detergent.

21. The use of a merocyanine dye in combination with a detergent as a poly(amino acid) stain in a cell-free environment.

22. The use of claim 21 wherein the merocyanine dye is as defined in any of Claims 3 to 14 or an appropriate combination thereof.

23. The use of claim 21 wherein the merocyanine dye is as defined in claim 5.

24. The use of claim 21 wherein the merocyanine dye has the formula 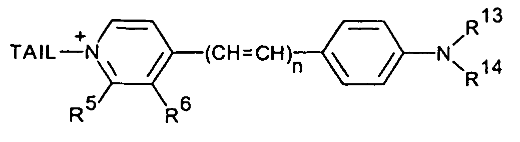 or 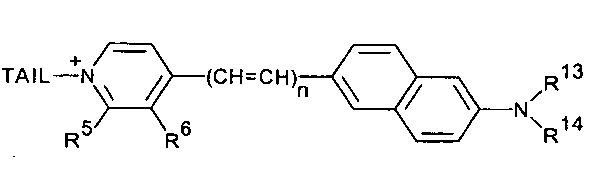 wherein
R⁵ and R⁶ are independently H, Cl, F, C₁-C₆ alkyl, C₁-C₆ perfluoroalkyl, C₁-C₆ alkoxy, amino, or amino substituted by 1-2 C₁-C₆ alkyls; or R⁵ and R⁶ taken in combination form a fused 6-membered aromatic ring that is optionally substituted by one or more substituents which may be the same or different and are selected from by Cl, F, C₁-C₆ alkyl, C₁-C₆ perfluoroalkyl, C₁-C₆ alkoxy, amino, or amino substituted by 1-2 C₁-C₆ alkyls;
TAIL is -CH₃, or CH₂CH₃; or TAIL is a C₃-C₂₂ alkyl chain that is linear or branched, saturated or unsaturated, that is optionally substituted one or more times by hydroxy, carboxy, sulfo, amino, amino substituted by 1-2 C₁-C₆ alkyls, or ammonium substituted by 1-3 C₁-C₆ alkyls;
n = 1 or 2; and
R¹³ and R¹⁴ are independently C₁-C₁₈ alkyl that is linear, branched, saturated or unsaturated, and is optionally substituted one or more times by F.

25. The use of any of claims 21 to 24 wherein the detergent is used at a concentration below its critical micelle concentration.

26. The use of any of claims 21 to 25 wherein the merocyanine dye is used as a non-selective dye to stain any poly(amino acids) in the sample being stained, subject to differing sensitivities of different poly(amino acids).

## Patentansprüche

1. Ein Verfahren zum Nachweis einer Poly(aminosäure) in einem Probengemisch, das die folgenden Schritte umfasst:
a) Herstellen, aus dem Probengemisch und einem Anfärbegemisch, das einen oder mehr als einen Merocyanin-Farbstoff beinhaltet, eines zellfreien kombinierten Gemischs, das die Poly(aminosäure) (falls vorhanden), den einen oder mehr als einen Merocyanin-Farbstoff und ein Detergenz beinhaltet;
b) Inkubieren des kombinierten Gemischs, damit dem Farbstoff in dem Anfärbegemisch die Verbindung mit jeder Poly(aminosäure) in dem Probengemisch zur Bildung eines Farbstoff-Poly(aminosäure)-Komplexes gestattet wird, der eine nachweisbare optische Reaktion bei Belichtung liefert;
c) Belichten eines Produkts, das unter Verwendung von Schritt (c) erhalten wurde, z. B. Farbstoff-Poly(aminosäure)-Komplex; und
d) Erfassen der optischen Reaktion.

2. Ein Verfahren nach Anspruch 1, wobei zwischen den Schritten a) und b) das Probengemisch oder das kombinierte Gemisch erwärmt wird.

3. Ein Verfahren nach Anspruch 1 oder 2, wobei das Detergenz gleichzeitig mit oder als Teil des Probengemischs oder des Anfärbegemischs zugegeben wird, oder nach Kombination des Proben- und des Anfärbegemischs zugegeben wird.

4. Ein Verfahren nach Anspruch 1, 2 oder 3, wobei jeder Merocyanin-Farbstoff unabhängig die folgende Formel aufweist:
Q-B-M
wobei folgendes gilt: Q ist 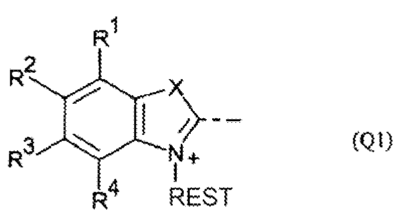 oder 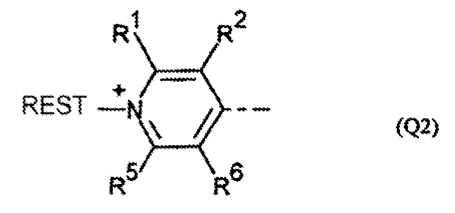 oder 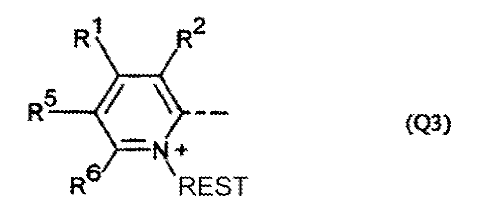 wobei folgendes gilt:
R¹, R², R³ und R⁴ sind unabhängig H, Cl, F, C₁-C₆-Alkyl, C₁-C₆-Perfluoralkyl, C₁-C₆-Alkoxy, Amino oder Amino, substituiert mit 1-2 C₁-C₆-Alkylgruppen,
R⁵ und R⁶ sind unabhängig H, Cl, F, C₁-C₆-Alkyl, C₁-C₆-Perfluoralkyl, C₁-C₆-Alkoxy, Amino oder Amino, substituiert mit 1-2 C₁-C₆-Alkylgruppen, oder Phenyl; oder R⁵ und R⁶ bilden zusammen einen verknüpften 6-gliedrigen aromatischen Ring, der wahlweise einfach oder mehrfach mit Substituenten substituiert ist, die gleich oder verschieden sein können und aus Cl, F, C₁-C₆-Alkyl, C₁-C₆-Perfluoralkyl, C₁-C₆-Alkoxy, Amino und Amino, substituiert mit 1-2 C₁-C₆-Alkylgruppen, ausgewählt sind, oder der verknüpfte 6-gliedrige aromatische Ring ist wahlweise mit einem weiteren verknüpften 6-gliedrigen aromatischen Ring substituiert, der wahlweise mit Substituenten substituiert ist, die gleich oder verschieden sein können und aus Cl, F, C₁-C₆-Alkyl, C₁-C₆-Perfluoralkyl, C₁-C₆-Alkoxy, Amino und Amino, substituiert mit 1-2 C₁-C₆-Alkylgruppen, ausgewählt sind;
X ist -S-, -O-, -NR⁷- oder -CR⁷R⁸-, wobei R⁷ und R⁸ unabhängig H, C₁-C₆-Alkyl oder Phenyl sind; oder R⁷ und R⁸ zusammen einen 5- oder 6-gliedrigen gesättigten Ring bilden;
REST ist an Q über ein Kohlenstoffatom angehängt und beinhaltet 1-22 Nicht-Wasserstoffatome, die C, O, N oder S sind, derart, dass jedes Heteroatom von beliebigen benachbarten Heteroatomen durch mindestens zwei Kohlenstoffatome getrennt ist; und ferner derart, dass der REST aus Kohlenstoff-Kohlenstoff-Bindungen (C-C), Etherbindungen (C-O-C), Thioetherbindungen (C-S-C) oder Aminbindungen (C-NR⁹-C) besteht; wobei ein beliebiges Kohlenstoffatom in dem REST wahlweise mit Hydroxy, Carboxy, Sulfo, Amino oder Ammonium weiter substituiert ist; und wobei eine beliebige Aminbindung, Amino oder Ammonium in dem REST wahlweise mit einem R⁹ substituiert ist, das ein C₂-C₆-Alkyl ist, das wahlweise mit Hydroxy, Carboxy, Sulfo, Amino, Amino, substituiert mit 1-2 C₁-C₆-Alkylgruppen, oder Ammonium, substituiert mit 1-3 C₁-C₆-Alkylgruppen, weiter substituiert ist, oder die N-Atome entweder ein oder zwei gesättigte 5- oder 6-gliedrige Ringe zusammen mit weiteren C- oder N-Atomen in dem REST bilden;
B ist
-(CR⁷=CR⁸)ₙ-
wobei R⁷ und R⁸ wie vorstehend definiert sind;
n = 1, 2 oder 3;
und M ist 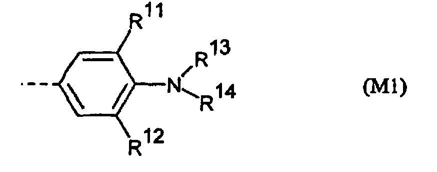 oder 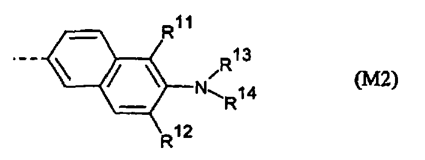 wobei folgendes gilt:
R¹¹ und R¹² sind unabhängig H, F, Cl oder -CH₃;
R¹³ und R¹⁴ sind unabhängig C₁-C₁₈-Alkyl, das gerade, verzweigt, gesättigt oder ungesättigt ist, und wahlweise einfach oder mehrfach mit F, Hydroxy oder C₁-C₆-Alkoxy substituiert ist; oder R¹³ und R¹⁴ zusammen einen 5- oder 6-gliedrigen gesättigten Ring bilden, der 0 oder 1 Sauerstoff-Heteroatome beinhaltet; oder R¹³ zusammen mit R¹¹ und R¹⁴ zusammen mit R¹² unabhängig -(CH₂)₂- oder -(CH₂)₃- sind;
oder M ist 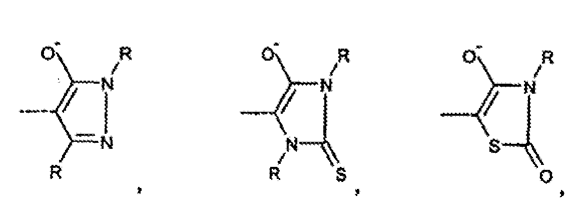 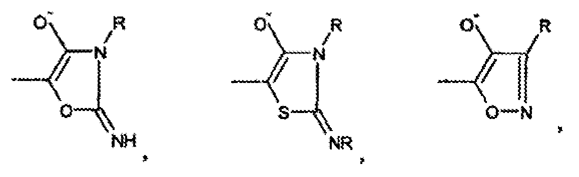 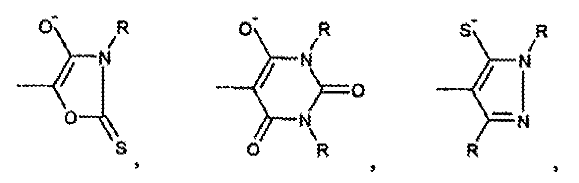 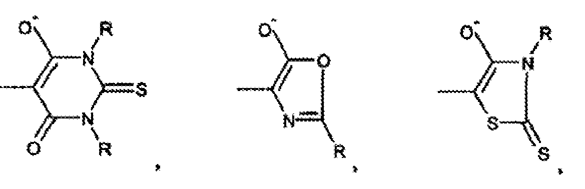 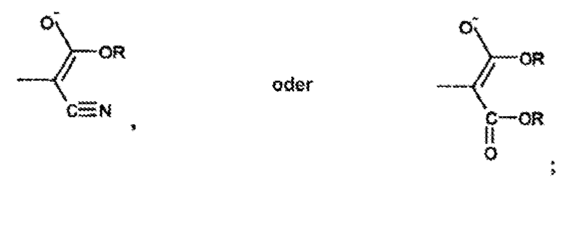 wobei folgendes gilt: R ist unabhängig H, Phenyl, Sulfophenyl, C₁-C₆-Alkyl oder C₁-C₆-Alkyl, substituiert mit Carboxy.

5. Ein Verfahren nach Anspruch 1 oder 2, wobei das Anfärbegemisch einen oder mehr als einen Styrol-Farbstoff beinhaltet, der unabhängig die Formel Q-B-M aufweist, wobei Q und B wie in Anspruch 4 definiert sind, und M die Formel M1 oder M2 aufweist, wie in Anspruch 4 definiert ist.

6. Ein Verfahren nach Anspruch 4 oder 5, wobei das Anfärbegemisch einen Farbstoff beinhaltet, wobei:
(i) M die Formel M1 aufweist; oder
(ii) M die Formel M2 aufweist.

7. Ein Verfahren nach Anspruch 4 oder 5, wobei das Anfärbegemisch einen Farbstoff beinhaltet, der die folgende Formel aufweist:

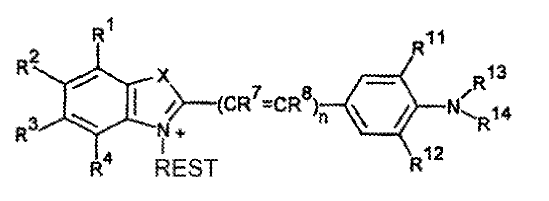

8. Ein Verfahren nach Anspruch 4 oder 5, wobei das Anfärbegemisch einen Farbstoff beinhaltet, der die folgende Formel aufweist:

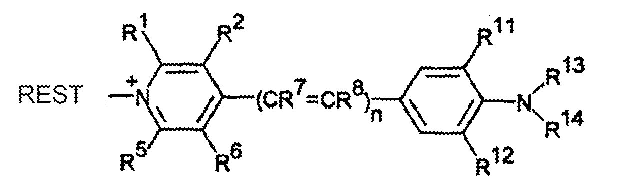

9. Ein Verfahren nach Anspruch 4 oder 5, wobei das Anfärbegemisch einen Farbstoff beinhaltet, der die folgende Formel aufweist:

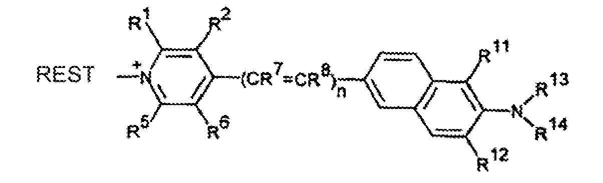

10. Ein Verfahren nach Anspruch 4 oder 5, wobei das Anfärbegemisch einen Farbstoff beinhaltet, der die folgende Formel aufweist: 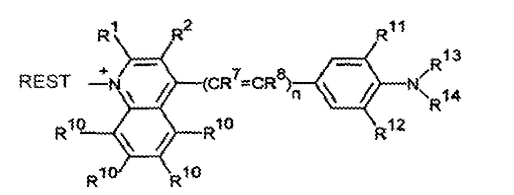 wobei folgendes gilt: jedes R¹⁰ ist unabhängig H, Cl, F, C₁-C₆-Alkyl, C₁-C₆-Perfluoralkyl, C₁-C₆-Alkoxy, Amino, Amino, substituiert mit 1-2 C₁-C₆-Alkylgruppen; oder beliebige zwei benachbarte R¹⁰-Substituenten bilden zusammen einen verknüpften 6-gliedrigen aromatischen Ring, der wahlweise mit Substituenten substituiert ist, die gleich oder verschieden sein können und aus Cl, F, C₁-C₆-Alkyl, C₁-C₆-Perfluoralkyl, C₁-C₆-Alkoxy, Amino und Amino, substituiert mit 1-2 C₁-C₆-Alkylgruppen, ausgewählt sind.

11. Ein Verfahren nach einem der Ansprüche 4 bis 10, wobei das Anfärbegemisch einen Farbstoff beinhaltet, wobei folgendes gilt:
REST ist -CH₃ oder -CH₂CH₃; oder REST ist eine C₃-C₂₂-Alkylkette, die gerade oder verzweigt, gesättigt oder ungesättigt ist, die wahlweise einfach oder mehrfach mit Hydroxy, Carboxy, Sulfo, Amino, Amino, substituiert mit 1-2 C₁-C₆-Alkylgruppen, oder Ammonium, substituiert mit 1-3 C₁-C₆-Alkylgruppen, substituiert ist.

12. Ein Verfahren nach Anspruch 4 oder 5, wobei das Anfärbegemisch einen Farbstoff beinhaltet, wobei folgendes gilt:
M weist die Formel M1 oder M2 auf;
X ist O oder S;
B ist -(CH=CH)ₙ-;
n = 1 oder 2;
R¹¹ und R¹² sind jeweils H;
R¹³ und R¹⁴ sind unabhängig C₄-C₈-Alkyl, und
REST ist Sulfopropyl, Sulfobutyl, Aminopropyl oder Aminobutyl, wobei die Amine 1-3 mal mit einer beliebigen Kombination aus H, Methyl oder Ethyl substituiert sind.

13. Ein Verfahren nach Anspruch 12, wobei das Anfärbegemisch einen Farbstoff beinhaltet, wobei folgendes gilt:
R¹, R², R³ und R⁴ sind jeweils H;
R⁵ und R⁶ sind jeweils H; und
der Farbstoff ist in dem kombinierten Gemisch bei einer Konzentration von 0,10 µM - 10 µM für eine fluoreszierende optische Reaktion oder 10 µM - 100 µM für eine colorimetrische optische Reaktion vorhanden; und
die Poly(aminosäure) weist eine Molmasse von 500 - 200.000 Dalton auf.

14. Ein Verfahren nach einem der Ansprüche 4 bis 6 oder 8 bis 10, wobei
(i) das Anfärbegemisch einen Farbstoff beinhaltet, wobei Q Q1 ist;
(ii) das Anfärbegemisch einen Farbstoff beinhaltet, wobei Q Q2 ist; oder
(iii) das Anfärbegemisch einen Farbstoff beinhaltet, wobei Q Q3 ist.

15. Ein Verfahren nach einem der Ansprüche 1 bis 14, das ferner folgendes umfasst: Transferieren des Probengemischs auf eine feste oder halbfeste Matrix vor oder nach der Kombination mit dem Anfärbegemisch.

16. Ein Verfahren nach einem der Ansprüche 1 bis 15, das ferner folgendes umfasst: Entfernen, Zerstören oder Dispergieren jeder biologischen Membran, die in dem Probengemisch vorhanden ist, unter der kritischen Mizellenkonzentration; und wahlweise ferner folgendes umfasst:
(i) Quantifizieren der Poly(aminosäure) durch Messung der nachweisbaren optischen Reaktion und Vergleichen der Messung mit einem Standard; und/oder
(ii) elektrophoretisches Trennen des Probengemischs vor, nach oder während es mit dem Anfärbegemisch kombiniert wird; und/oder
(iii) Zugeben von einem weiteren Reagenz zu dem Probengemisch, dem Anfärbegemisch oder dem kombinierten Gemisch.

17. Ein Verfahren nach einem der Ansprüche 1 bis 15, das ferner folgendes umfasst: Erwärmen des Probengemischs oder des kombinierten Gemischs; und wahlweise ferner folgendes umfasst:
(i) Quantifizieren der Poly(aminosäure) durch Messung der nachweisbaren optischen Reaktion und Vergleichen der Messung mit einem Standard; und/oder
(ii) elektrophoretisches Trennen des Probengemischs vor, nach oder während es mit dem Anfärbegemisch kombiniert wird; und/oder
(iii) Zugeben von einem weiteren Reagenz zu dem Probengemisch, dem Anfärbegemisch oder dem kombinierten Gemisch.

18. Ein Verfahren nach einem der Ansprüche 1 bis 17, wobei die nachweisbare optische Reaktion eine Fluoreszenzreaktion ist.

19. Ein Verfahren nach einem der Ansprüche 1 bis 18, wobei das Probengemisch eine Poly(aminosäure) beinhaltet und der Schritt (c) die Belichtung eines Komplexes ist, der von der Poly(aminosäure) und dem Farbstoff in dem Schritt (b) gebildet wurde, und der Schritt (d) die Erfassung der optischen Reaktion des Komplexes ist.

20. Eine Kombination, die folgendes umfasst:
a) einen oder mehr als einen Styrol-Farbstoff mit der Formel
Q-B-M
wobei folgendes gilt: Q ist Q1, Q2 oder Q3, wie in Anspruch 4 definiert ist;
B ist
-(CR⁷=CR⁸)ₙ-
wobei R⁷ und R⁸ wie in Anspruch 1 definiert sind;
n = 1, 2 oder 3;
und M ist M1 oder M2, wie in Anspruch 4 definiert ist;
b) ein Detergenz; und
c) eine Poly(aminosäure),
in einer zellfreien wässrigen Lösung, wobei das Detergenz bei einer Konzentration unterhalb der kritischen Mizellenkonzentration für das Detergenz vorhanden ist.

21. Die Verwendung eines Merocyanin-Farbstoffs in Kombination mit einem Detergenz als ein Poly(aminosäure)-Anfärber in einer zellfreien Umgebung.

22. Die Verwendung nach Anspruch 21, wobei der Merocyanin-Farbstoff wie in einem der Ansprüche 3 bis 14 oder einer geeigneten Kombination davon definiert ist.

23. Die Verwendung nach Anspruch 21, wobei der Merocyanin-Farbstoff wie in Anspruch 5 definiert ist.

24. Die Verwendung nach Anspruch 21, wobei der Merocyanin-Farbstoff die folgende Formel aufweist: 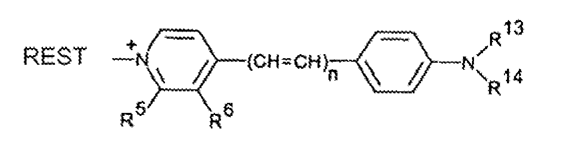 oder 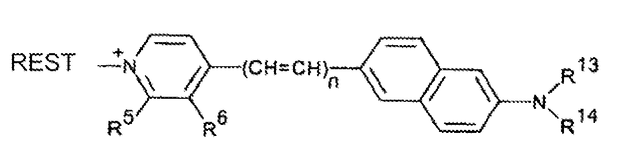 wobei folgendes gilt:
R⁵ und R⁶ sind unabhängig H, Cl, F, C₁-C₆-Alkyl, C₁-C₆-Perfluoralkyl, C₁-C₆-Alkoxy, Amino oder Amino, substituiert mit 1-2 C₁-C₆-Alkylgruppen, oder R⁵
und R⁶ bilden zusammen einen verknüpften 6-gliedrigen aromatischen Ring, der wahlweise mit einem oder mehr als einem Substituenten substituiert ist, der gleich oder verschieden sein kann und aus Cl, F, C₁-C₆-Alkyl, C₁-C₆-Perfluoralkyl, C₁-C₆-Alkoxy, Amino oder Amino, substituiert mit 1-2 C₁-C₆-Alkylgruppen, ausgewählt ist;
REST ist -CH₃ oder -CH₂CH₃; oder REST ist eine C₃-C₂₂-Alkylkette, die gerade oder verzweigt, gesättigt oder ungesättigt ist, die wahlweise einfach oder mehrfach mit Hydroxy, Carboxy, Sulfo, Amino, Amino, substituiert mit 1-2 C₁-C₆-Alkylgruppen, oder Ammonium, substituiert mit 1-3 C₁-C₆-Alkylgruppen, substituiert ist;
n = 1 oder 2; und
R¹³ und R¹⁴ sind unabhängig C₁-C₁₈-Alkyl, das gerade, verzweigt, gesättigt oder ungesättigt ist, und wahlweise einfach oder mehrfach mit F substituiert ist.

25. Die Verwendung nach einem der Ansprüche 21 bis 24, wobei das Detergenz bei einer Konzentration unterhalb seiner kritischen Mizellenkonzentration verwendet wird.

26. Die Verwendung nach einem der Ansprüche 21 bis 25, wobei der Merocyanin-Farbstoff als ein nicht-selektiver Farbstoff zum Anfärben jeder Poly(aminosäure) in der anzufärbenden Probe verwendet wird und einer verschiedenen Empfindlichkeit für verschiedene Poly(aminosäure)n unterliegt.

## Revendications

1. Procédé de détection pour un poly(acide aminé) dans un mélange échantillon, comprenant les étapes consistant à :
a) préparer, à partir du mélange échantillon et d'un mélange colorant qui contient un ou plusieurs colorants mérocyanines, un mélange combiné exempt de cellules contenant le poly(acide aminé) (s'il est présent), le ou les colorants mérocyanines et un détergent ;
b) faire incuber le mélange combiné pour amener le colorant dans le mélange colorant à s'associer avec tout poly(acide aminé) dans le mélange échantillon pour former un complexe colorant-poly(acide aminé) qui donne une réponse optique détectable lors de l'éclairement ;
c) éclairer le produit obtenu à l'aide de l'étape (c), par exemple un complexe colorant-poly(acide aminé) ; et
d) observer la réponse optique.

2. Procédé selon la revendication 1, dans lequel, entre les étapes a) et b), le mélange échantillon ou le mélange combiné est chauffé.

3. Procédé selon l'une des revendications 1 ou 2, dans lequel le détergent est ajouté simultanément à ou comme partie du mélange échantillon ou du mélange colorant, ou est ajouté après que les mélanges échantillon et colorant aient été combinés.

4. Procédé, selon l'une de revendications 1, 2 ou 3, dans lequel chaque colorant mérocyanine a indépendamment la formule :
Q-B-M
où Q est : 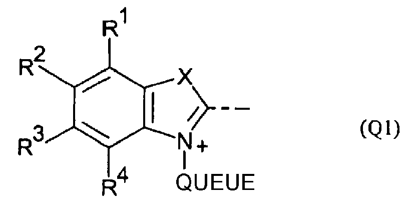 ou 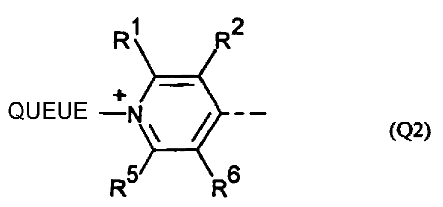 ou 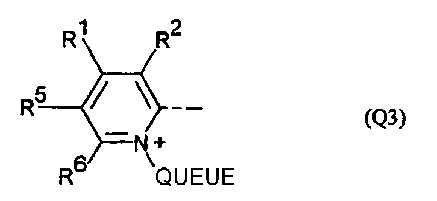 où
- R¹, R², R³ et R⁴ représentent indépendamment H, Cl, F, alkyle en C₁-C₆, perfluoroalkyle en C₁-C₆, alcoxy en C₁-C₆, amino ou amino substitué par 1-2 alkyles en C₁-C₆ ;
- R⁵ et R⁶ représentent indépendamment H, Cl, F, alkyle en C₁-C₆, perfluoroalkyle en C₁-C₆, alcoxy en C₁-C₆, amino ou amino substitué par 1-2 alkyles en C₁-C₆ ou phényle ; ou R⁵ et R⁶ pris en combinaison forment un noyau aromatique à 6 chaînons fusionné qui est facultativement substitué une ou plusieurs fois par des substituants qui peuvent être identiques ou différents et sont choisis parmi Cl, F, alkyle en C₁-C₆, perfluoroalkyle en C₁-C₆, alcoxy en C₁-C₆, amino et amino substitué par 1-2 alkyles en C₁-C₆,
ou ledit noyau aromatique à 6 chaînons fusionné est facultativement substitué par un noyau aromatique à 6 chaînons fusionné supplémentaire qui est facultativement substitué par des substituants qui peuvent être identiques ou différents et sont choisis parmi Cl, F, alkyle en C₁-C₆, perfluoroalkyle en C₁-C₆, alcoxy en C₁-C₆, amino et amino substitué par 1-2 alkyles en C₁-C₆ ;
- X représente -S-, -O-, -NR⁷- ou -CR⁷R⁸-, où R⁷ et R⁸ sont indépendamment H, alkyle en C₁-C₆ ou phényle ; ou R⁷ et R⁸ pris en combinaison complètent un noyau saturé à 5 ou 6 chaînons ;
- QUEUE est attachée à Q par un atome de carbone et contient 1-22 atomes non hydrogène, qui sont C, 0, N ou S, de telle sorte que chaque hétéroatome est séparé de tous hétéroatomes adjacents par au moins deux atomes de carbone ; et en outre par le fait que QUEUE est composé de liaisons carbone-carbone (C-C), liaisons éther (C-OC), liaisons thioéther (C-S-C) ou liaisons amine (C-NR⁹-C) ; où tout atome de carbone dans QUEUE est facultativement encore substitué par hydroxy, carboxy, sulfo, amino ou ammonium ; et où toute liaison amine, amino ou ammonium dans QUEUE est facultativement substituée par R⁹ qui est un alkyle en C₂-C₆ qui est facultativement encore substitué par hydroxy, carboxy, sulfo, amino, amino substitué par 1-2 alkyles en C₁-C₆,
ou ammonium substitué par 1-3 alkyles en C₁-C₆, ou lesdits atomes de N forment soit un soit deux cycles à 5 ou 6 chaînons saturés en combinaison avec des atomes de C ou N supplémentaires dans QUEUE ;
- B représente
- (CR⁷=CR⁸)ₙ-
où R⁷ et R⁸ sont tels que définis précédemment ;
n = 1, 2 ou 3 ;
et M représente : 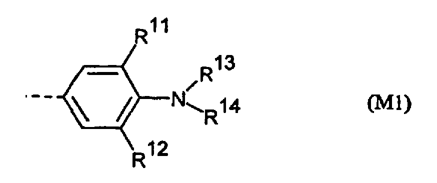 ou 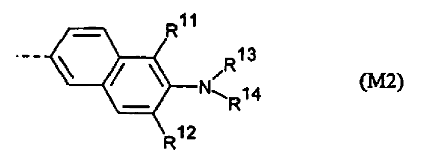 où :
- R¹¹ et R¹² représentent indépendamment H, F, Cl ou -CH₃ ;
- R¹³ et R¹⁴ représentent indépendamment alkyle en C₁-C₁₈ qui est linéaire, ramifié, saturé ou insaturé, et est facultativement substitué une ou plusieurs fois par F, hydroxy ou alcoxy en C₁-C₆ ; ou R¹³ et R¹⁴ pris en combinaison forment un noyau saturé à 5 ou 6 chaînons contenant 0 ou 1 hétéroatome d'oxygène ; ou R¹³ pris en combinaison avec R¹¹ et R¹⁴ pris en combinaison avec R¹² représentent indépendamment -(CH₂)₂- ou -(CH₂)₃- ;
ou M représente : 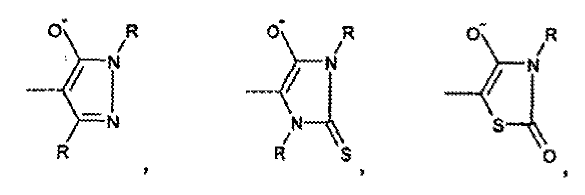 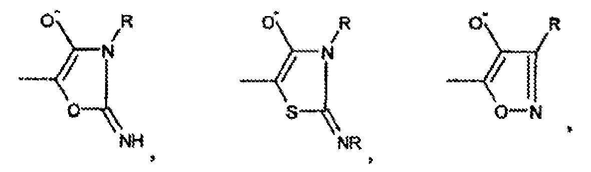 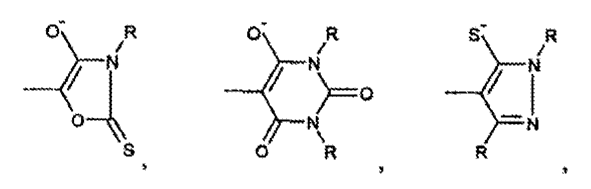 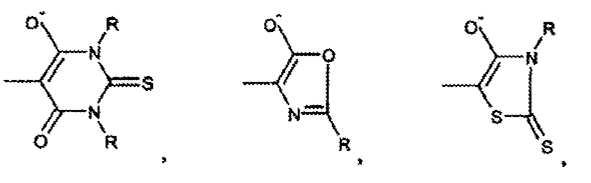 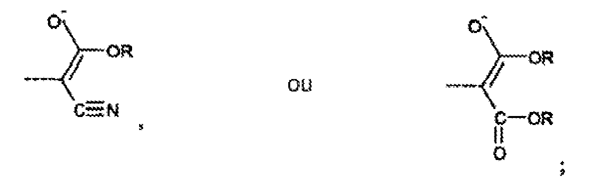 où
R représente indépendamment H, phényle, sulfophényle, alkyle en C₁-C₆ ou alkyle en C₁-C₆ substitué par carboxy.

5. Procédé selon l'une des revendications 1 ou 2, dans lequel le mélange colorant contient un ou plusieurs colorants styrylés ayant indépendamment la formule Q-B-M, où Q et B sont tels que définis dans la revendication 4 et M a la formule M1 ou M2 telle que définie dans la revendication 4.

6. Procédé selon l'une des revendications 4 ou 5, dans lequel le mélange colorant contient un colorant dans lequel :
(i) M a la formule M1 ; ou
(ii) M a la formule M2.

7. Procédé selon l'une des revendications 4 ou 5, dans lequel le mélange colorant contient un colorant qui a la formule :

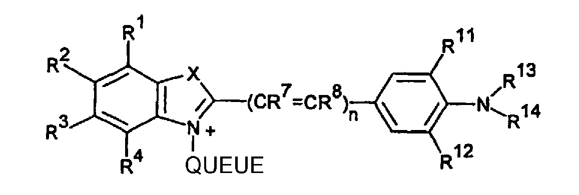

8. Procédé selon l'une des revendications 4 ou 5, dans lequel le mélange colorant contient un colorant qui a la formule :

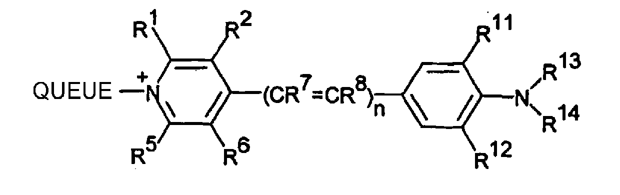

9. Procédé selon l'une des revendications 4 ou 5, dans lequel le mélange colorant contient un colorant qui a la formule :

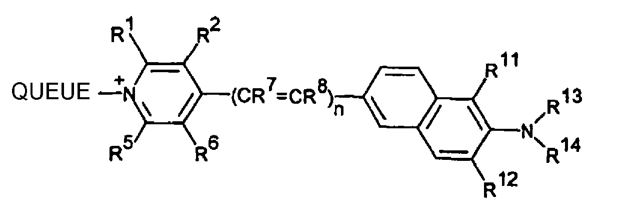

10. Procédé selon l'une des revendications 4 ou 5, dans lequel le mélange colorant contient un colorant qui a la formule : 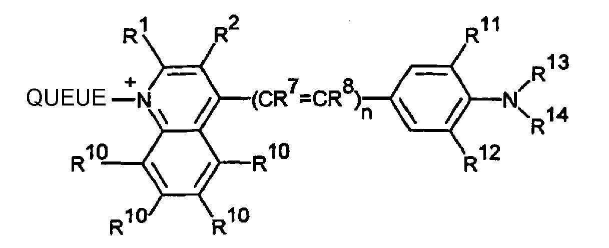 où les R¹⁰ représentent chacun indépendamment H, Cl, F, alkyle en C₁-C₆, perfluoroalkyle en C₁-C₆, alcoxy en C₁-C₆, amino, amino substitué par 1-2 alkyles en C₁-C₆ ou n'importe quels deux substituants R¹⁰ adjacents, lorsqu'ils sont pris en combinaison, forment un noyau aromatique à 6 chaînons fusionné qui est facultativement substitué par des substituants qui peuvent être identiques ou différents et sont choisis parmi Cl, F, alkyle en C₁-C₆, perfluoroalkyle en C₁-C₆, alcoxy en C₁-C₆, amino et amino substitué par 1-2 alkyles en C₁-C₆.

11. Procédé selon l'une quelconque des revendications 4 à 10, dans lequel le mélange colorant contient un colorant dans lequel :
QUEUE représente -CH₃ ou CH₂CH₃ ; ou QUEUE représente une chaîne alkyle en C₃-C₂₂ qui est linéaire ou ramifiée, saturée ou insaturée, qui est facultativement substituée une ou plusieurs fois par hydroxy, carboxy, sulfo, amino, amino substitué par 1-2 alkyles en C₁-C₆ ou ammonium substitué par 1-3 alkyles en C₁-C₆.

12. Procédé selon l'une des revendications 4 ou 5, dans lequel le mélange colorant contient un colorant dans lequel :
- M a la formule M1 ou M2 ;
- X représente 0 ou S ;
- B représente -(CH=CH)ₙ- ;
- n = 1 ou 2 ;
- R¹¹ et R¹² représentent chacun H ;
- R¹³ et R¹⁴ représentent indépendamment alkyle en C₄-C₈ ; et
- QUEUE représente sulfopropyle, sulfobutyle, aminopropyle
ou aminobutyle, dont les amines sont substituées 1-3 fois par toute combinaison de H, méthyle ou éthyle.

13. Procédé selon la revendication 12, dans lequel le mélange colorant contient un colorant dans lequel :
- R¹, R², R³ et R⁴ représentent chacun H ;
- R⁵ et R⁶ représentent chacun H ; et
- ledit colorant est présent dans le mélange combiné à une concentration de 0,10 µM-10 µM pour une réponse optique fluorescente ou 10 µM-100 µM pour une réponse optique colorimétrique ; et
- ledit poly(acide aminé) a une masse moléculaire de 500-200 000 daltons.

14. Procédé selon l'une quelconque des revendications 4 à 6 ou 8 à 10, dans lequel :
(i) le mélange colorant contient un colorant dans lequel Q représente Q1 ;
(ii) le mélange colorant contient un colorant dans lequel Q représente Q2 ; ou
(iii) le mélange colorant contient un colorant dans lequel Q représente Q3.

15. Procédé selon l'une quelconque des revendications 1 à 14, comprenant en outre le transfert du mélange échantillon dans une matrice solide ou semi-solide avant ou après combinaison avec le mélange colorant.

16. Procédé selon l'une quelconque des revendications 1 à 15, comprenant en outre les opérations consistant à retirer, détruire ou disperser au-dessous de la concentration de micelles critique toutes membranes biologiques qui sont présentes dans le mélange échantillon ; et comprenant en outre facultativement les opérations consistant à :
(i) quantifier ledit poly(acide aminé) par mesure de ladite réponse optique détectable et comparer ladite mesure avec un étalon ; et/ou
(ii) séparer par voie électrophorétique le mélange échantillon avant, après ou pendant qu'il est combiné avec le mélange colorant ; et/ou
(iii) ajouter un réactif supplémentaire au mélange échantillon, au mélange colorant ou au mélange combiné.

17. Procédé selon l'une quelconque des revendications 1 à 15, comprenant en outre les opérations consistant à chauffer le mélange échantillon ou le mélange combiné ; et facultativement comprenant en outre les opérations consistant à :
(i) quantifier ledit poly(acide aminé) par mesure de ladite réponse optique détectable et comparer ladite mesure avec un étalon ; et/ou
(ii) séparer par voie électrophorétique le mélange échantillon avant, après ou pendant qu'il est combiné avec le mélange colorant ; et/ou
(iii) ajouter un réactif supplémentaire au mélange échantillon, au mélange colorant ou au mélange combiné.

18. Procédé selon l'une quelconque des revendications 1 à 17, dans lequel ladite réponse optique détectable est une réponse de fluorescence.

19. Procédé selon l'une quelconque des revendications 1 à 18, dans lequel le mélange échantillon contient un poly(acide aminé) et l'étape (c) est un éclairement d'un complexe formé par le poly(acide aminé) et le colorant à l'étape (b), l'étape (d) étant une observation de la réponse optique du complexe.

20. Combinaison comprenant :
a) un ou plusieurs colorants styrylés ayant la formule
Q-B-M
où Q représente Q1, Q2 ou Q3 tel que défini dans la revendication 4 ;
B est
-(CR⁷=CR⁸)ₙ-
où R⁷ et R⁸ sont tels que définis dans la revendication 1
n = 1, 2 ou 3 ;
et M représente M1 ou M2 tel que défini dans la revendication 4 ;
b) un détergent ; et
c) un poly(acide aminé),
dans une solution aqueuse exempte de cellules où ledit détergent est présent à une concentration inférieure à la concentration critique de micelles pour ce détergent.

21. Utilisation d'un colorant mérocyanine en combinaison avec un détergent en tant que colorant de poly(acide aminé) dans un environnement exempt de cellules.

22. Utilisation selon la revendication 21, dans laquelle le colorant mérocyanine est tel que défini à l'une quelconque des revendications 3 à 14 ou une combinaison appropriée de celui-ci.

23. Utilisation selon la revendication 21, dans laquelle le colorant mérocyanine est tel que défini à la revendication 5.

24. Utilisation selon la revendication 21, dans laquelle le colorant mérocyanine a la formule : 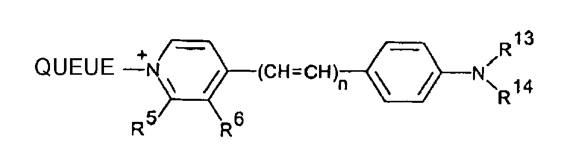 ou 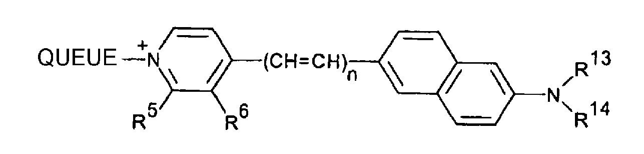 où
- R⁵ et R⁶ représentent indépendamment H, Cl, F, alkyle en C₁-C₆, perfluoroalkyle en C₁-C₆, alcoxy en C₁-C₆, amino ou amino substitué par 1-2 alkyles en C₁-C₆ ; ou R⁵ et R⁶ pris en combinaison forment un noyau aromatique à 6 chaînons fusionné qui est facultativement substitué par un ou plusieurs substituants qui peuvent être identiques
ou différents et sont choisis parmi Cl, F, alkyle en C₁-C₆, perfluoroalkyle en C₁-C₆, alcoxy en C₁-C₆, amino ou amino substitué par 1-2 alkyles en C₁-C₆ ;
- QUEUE représente -CH₃ ou CH₂CH₃ ; ou QUEUE représente une chaîne alkyle en C₃-C₂₂ qui est linéaire ou ramifié, saturé ou insaturé, qui est facultativement substitué une
ou plusieurs fois par hydroxy, carboxy, sulfo, amino, amino substitué par 1-2 alkyles en C₁-C₆ ou ammonium substitué par 1-3 alkyles en C₁-C₆ ;
- n = 1 ou 2 ; et
- R¹³ et R¹⁴ représentent indépendamment alkyle en C₁-C₁₈ qui est linéaire, ramifié, saturé ou insaturé, et est facultativement substitué une ou plusieurs fois par F.

25. Utilisation selon l'une quelconque des revendications 21 à 24, dans laquelle le détergent est utilisé à une concentration au-dessous de sa concentration de micelles critique.

26. Utilisation selon l'une quelconque des revendications 21 à 25, dans laquelle le colorant mérocyanine est utilisé comme colorant non sélectif pour colorer tous poly(acides aminés) dans l'échantillon qui est coloré, soumis à différentes sensibilités de différents poly(acides aminés).
